(19) Europäisches Patentamt
European Patent Office
Office européen des brevets

(11) EP 1 237 877 B1

(12) **EUROPÄISCHE PATENTSCHRIFT**

(45) Veröffentlichungstag und Bekanntmachung des Hinweises auf die Patenterteilung:
**27.07.2005 Patentblatt 2005/30**

(21) Anmeldenummer: **00984982.9**

(22) Anmeldetag: **08.11.2000**

(51) Int Cl.[7]: **C07D 235/00**

(86) Internationale Anmeldenummer:
**PCT/EP2000/010996**

(87) Internationale Veröffentlichungsnummer:
**WO 2001/034572 (17.05.2001 Gazette 2001/20)**

(54) **CARBOXAMID-SUBSTITUIERTE BENZIMIDAZOL-DERIVATE, VERFAHREN ZU IHRER HERSTELLUNG UND IHRE VERWENDUNG ALS TRYPTASE-INHIBITOREN**

CARBOXAMIDE-SUBSTITUTED BENZIMIDAZOL DERIVATIVES, METHODS FOR THE PRODUCTION THEREOF AND THEIR USE AS MEDICAMENTS

DERIVE DE BENZIMIDAZOLE SUBSTITUES PAR CARBOXAMIDE, LEUR PROCEDE DE PRODUCTION ET LEUR UTILISATION COMME MEDICAMENTS

(84) Benannte Vertragsstaaten:
**AT BE CH CY DE DK ES FI FR GB GR IE IT LI LU MC NL PT SE TR**

(30) Priorität: **10.11.1999 DE 19953899**

(43) Veröffentlichungstag der Anmeldung:
**11.09.2002 Patentblatt 2002/37**

(73) Patentinhaber: **Boehringer Ingelheim Pharma GmbH & Co.KG**
**55218 Ingelheim am Rhein (DE)**

(72) Erfinder:
- **ANDERSKEWITZ, Ralf**
  **55411 Bingen (DE)**
- **BRAUN, Christine**
  **CH-6512 Giubiasco (CH)**
- **BRIEM, Hans**
  **55218 Ingelheim (DE)**
- **DISSE, Bernd**
  **55127 Mainz (DE)**
- **HOENKE, Christoph**
  **55218 Ingelheim (DE)**
- **JENNEWEIN, Hans, Michael**
  **65193 Wiesbaden (DE)**
- **SPECK, Georg**
  **55218 Ingelheim (DE)**

(56) Entgegenhaltungen:
**WO-A-00/08014** **WO-A-01/14342**
**WO-A-01/23359** **WO-A-99/40072**

- **CHEMICAL ABSTRACTS, vol. 132, no. 20, 15. Mai 2000 (2000-05-15) Columbus, Ohio, US; abstract no. 265201, SUZUKI, N. ET AL.: "Preparation of imidazole derivatives as gonadotropin-releasing hormone antagonists" XP002165472 & JP 2000 095767 A (TAKEDA CHEMICAL INDUSTRIES, LTD., JAPAN) 4. April 2000 (2000-04-04)**
- **CHEMICAL ABSTRACTS, vol. 133, no. 1, 3. Juli 2000 (2000-07-03) Columbus, Ohio, US; abstract no. 805, KUBO, K. ET AL.: "Benzimidazole derivatives as neovascularization inhibitors and pharmaceutical compositions containing them" XP002165473 & JP 2000 143635 A (TAKEDA CHEMICAL INDUSTRIES, LTD., JAPAN) 26. Mai 2000 (2000-05-26)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 237 877 B1

## Beschreibung

Die Erfindung betrifft Carboxamid-substituierte Benzimidazolderivate der allgemeinen Formel (I)

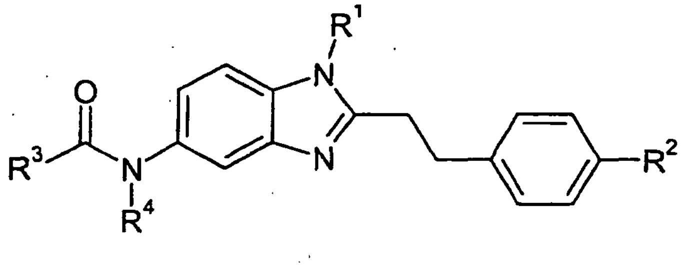

(I),

worin die Reste $R^1$, $R^2$, $R^3$ und $R^4$ die in den Ansprüchen und in der Beschreibung genannten Bedeutungen haben können, Verfahren zu deren Herstellung sowie die Verwendung von Carboxamid-substituierten Benzimidazolderivaten als Arzneimittel, insbesondere als Arzneimittel mit Tryptase-inhibierender Wirkung.

### Hintergrund der Erfindung

Benzimidazolderivate sind als Wirkstoffe mit wertvollen pharmazeutischen Eigenschaften aus dem Stand der Technik bekannt. So offenbart die Internationale Patentanmeldung WO 98/37075 neben anderen bicyclischen Heterocyclen auch Benzimidazole, die sich aufgrund einer thrombinhemmenden Wirkung zur Vorbeugung und Behandlung venöser und arterieller thrombotischer Erkrankungen wirksam einsetzen lassen. Die Internationale Patentanmeldung WO 00/08014 beschreibt antithrombotisch wirksame Verbindungen, die mit einem Disclaimer vom vorliegenden Anspruchsumfang ausgeschlossen sind.

Anders als der vorstehend beschriebenen und im Stand der Technik bereits bekannten Verwendung von Benzimidazolderivaten, liegt der vorliegenden Erfindung die Aufgabe zugrunde, neue Tryptase-Inhibitoren bereitzustellen, die aufgrund ihrer Tryptase-inhibierenden Eigenschaften zur Vorbeugung und Behandlung entzündlicher und/ oder allergischer Erkrankungen eingesetzt werden können.

### Detaillierte Beschreibung der Erfindung

Überraschenderweise wurde gefunden, daß Carboxamid-substituierte Benzimidazolderivate der allgemeinen Formel (I)

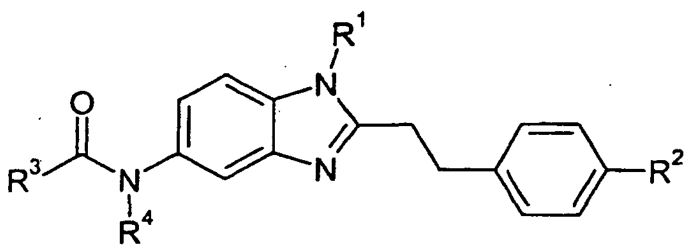

(I),

worin die Reste $R^1$, $R^2$, $R^3$ und $R^4$ die nachstehend genannten Bedeutungen tragen können, eine Tryptase-inhibierende Wirkung aufweisen und erfindungsgemäß zur Vorbeugung und Behandlung von Erkrankungen Verwendung finden können, in denen Tryptase-Inhibitoren einen therapeutischen Nutzen entfalten können.

Die vorliegende Erfindung betrifft Carboxamid-substituierte Benzimidazolderivate der allgemeinen Formel (I)

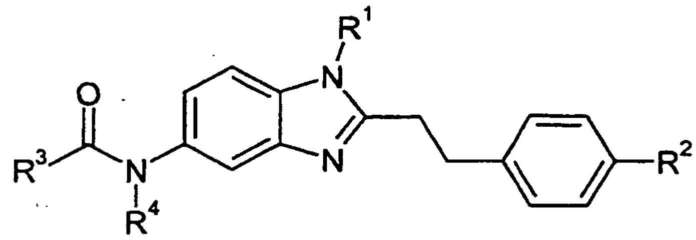

(I),

worin

R[1] ein Rest ausgewählt aus der Gruppe bestehend aus $C_1$-$C_6$-Alkyl, $C_2$-$C_6$-Alkenyl und $C_2$-$C_6$-Alkinyl, welcher gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -CO-$NR^5R^6$, -$NR^5R^6$ oder $C_1$-$C_4$-Alkoxy-phenoxy substituiert sein kann, oder
Phenyl-$C_1$-$C_4$-alkyl, welches gegebenenfalls ein-, zwei- oder dreifach durch einen oder mehrere der Reste Hydroxy, $C_1$-$C_4$-Alkoxy, Carboxy, Halogen, $C_1$-$C_4$-Alkoxycarbonyl oder $CF_3$ substituiert sein kann, oder ein direkt oder über eine $C_1$-$C_4$-Alkylen-Brücke verknüpfter 5- oder 6-gliedriger, gesättigter oder ungesättigter Heterocyclus, der ein oder zwei Heteroatome ausgewählt aus der Gruppe Sauerstoff, Stickstoff oder Schwefel enthalten kann und der gegebenenfalls durch $C_1$-$C_4$-Alkyl oder Benzyl substituiert sein kann;

$R^2$ -C(=NH)$NH_2$ oder -$CH_2$-$NH_2$;

$R^3$ ein $C_1$-$C_6$-Alkyl-, $C_1$-$C_6$-Hydroxyalkyl- oder $C_1$-$C_4$-Alkoxy-$C_1$-$C_4$-alkyl-Rest, der ein- oder zweifach durch einen, zwei oder drei der Reste -$NR^5R^6$, -C(=NH)$NH_2$ oder -NH-C(=NH)$NH_2$ substituiert sein kann, oder ein direkt, über eine $C_1$-$C_4$-Alkylen-Brücke oder eine $C_2$-$C_4$-Alkenylen-Brücke verknüpfter 5-, 6- oder 7-gliedriger, gesättigter oder ungesättigter Heterocyclus, der ein, zwei oder drei Heteroatome ausgewählt aus der Gruppe Sauerstoff, Stickstoff oder Schwefel enthalten kann, und der gegebenenfalls ein oder zweifach durch Hydroxy, $C_1$-$C_4$-Alkyl, -COO-$C_1$-$C_4$-Alkyl, -$CONH_2$, Benzyl, Diphenylmethyl, Phenyl oder Pyridylmethyl, Pyridyl substituiert sein kann, und wobei der Phenyl-Substituent ein-, zwei- oder dreifach durch einen oder mehrere der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$,substituiert sein kann, oder
Cyclopropyl, Cyclobutyl, Cyclopentyl oder Cyclohexyl, die jeweils ein- oder zweifach durch einen oder zwei der Reste -$NR^5R^6$, -C(=NH)$NH_2$ oder -NH-C(=NH)$NH_2$ substituiert sein können, oder
Phenyl-$C_1$-$C_4$-alkyl oder Naphthyl-$C_1$-$C_4$-alkyl, das an der Alkylenbrücke gegebenenfalls durch -$NR^5R^6$ substituiert sein kann und das am Phenylring jeweils ein- oder zweifach durch einen oder zwei der Reste -$NO_2$, -$NR^5R^6$, -$C_1$-$C_4$-Alkyl-$NR^5R^6$,
-C(=NH)$NH_2$ oder -NH-C(=NH)$NH_2$ substituiert sein kann,

$R^4$ Wasserstoff oder $C_1$-$C_6$-Alkyl, welches gegebenenfalls ein- oder zweifach durch einen oder zwei Reste ausgewählt aus der Gruppe Furanyl, Benzofuranyl, Thiophenyl, Benzothiophenyl, Anthracenyl, Phenyl, Pyridyl und Naphthyl substituiert sein kann, wobei die Substituenten Phenyl und Naphthyl ihrerseits jeweils ein-, zwei- oder dreifach durch einen oder mehrere der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$,
-NH($C_1$-$C_4$-alkyl), -N($C_1$-$C_4$-alkyl)$_2$, $NO_2$, Hydroxy, -$CF_3$,
-NHCO-$C_1$-$C_4$-alkyl, -COOH, -COO($C_1$-$C_4$-alkyl), -$CONH_2$,
-CONH($C_1$-$C_4$-alkyl), -CON($C_1$-$C_4$-alkyl)$_2$,
-CONH($C_1$-$C_4$-alkyl)-COO($C_1$-$C_4$-alkyl) und Phenyl-$C_1$-$C_6$-alkyl substituiert sein können;

$R^5$ und $R^6$ gleich oder verschieden, Wasserstoff, $C_1$-$C_4$-Alkyl, Phenyl, Pyridyl oder Benzyl, welches gegebenenfalls durch einen Rest ausgewählt aus der Gruppe Halogen, Halo-$C_1$-$C_4$-Alkyl, -OH, -$C_1$-$C_4$-Alkyl, -O-$C_1$-$C_4$-alkyl, -CO-O-$C_1$-$C_4$-alkyl, -$NO_2$, Phenyl, Pyrrolidin-1-yl, Piperidin-1-yl, -$NH_2$, -NH-$C_1$-$C_4$-alkyl, -N($C_1$-$C_4$-alkyl)$_2$ und -C(=NH)$NH_2$ substituiert sein kann,

bedeuten können,
mit der Maßgabe, dass Verbindungen der Formel 1, worin

$R^3$ eine $C_1$-$C_5$-Alkyl-, Phenyl-$C_1$-$C_3$-alkyl-, $C_3$-$C_6$-Cycloalkyl-, Pyridylgruppe, und
$R^4$ ein Wasserstoffatom, $C_1$-$C_5$ Alkyl oder Phenyl-$C_1$-$C_3$-alkylgruppe

bedeuten, ausgenommen sind, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß bevorzugt sind Verbindungen der allgemeinen Formel (I), worin

$R^1$ $C_1$-$C_6$-Alkyl, welches gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -CO-$NR^5R^6$, -$NR^5R^6$ oder $C_1$-$C_4$-Alkoxy-phenoxy substituiert sein kann, oder

$R^2$ -C(=NH)$NH_2$ oder-$CH_2$-$NH_2$;

$R^3$ ein $C_1$-$C_6$-Alkyl-Rest, der ein- oder zweifach durch einen oder zwei der Reste -$NR^5R^6$, -C(=NH)$NH_2$ oder -NH-C(=NH)$NH_2$ substituiert sein kann, oder
ein direkt oder über eine $C_1$-$C_4$-Alkylen-Brücke verknüpfter 5-, 6- oder 7-gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein oder zweifach durch Hydroxy, $C_1$-$C_4$-Alkyl, Benzyl, Phenyl oder Pyridyl substituiert sein kann, und wobei der Phenyl-Substituent durch einen der Reste ausgewählt aus der Gruppe $C_1$-$C_3$-Alkyl, $C_1$-$C_3$-Alkoxy, Halogen, Trifluormethyl und -$NH_2$, substituiert sein kann, oder
Cyclopropyl, Cyclopentyl oder Cyclohexyl, die jeweils ein- oder zweifach durch einen oder zwei der Reste -$NR^5R^6$, -C(=NH)$NH_2$ oder -NH-C(=NH)$NH_2$ substituiert sein können, oder
Phenyl-$C_1$-$C_4$-alkyl oder Naphthyl-$C_1$-$C_4$-alkyl, das an der Alkylenbrücke gegebenenfalls durch -$NR^5R^6$ substituiert sein kann und das am Phenylring jeweils ein- oder zweifach durch einen oder zwei der Reste -$NO_2$, -$NR^5R^6$, -$C_1$-$C_4$-Alkyl-$NR^5R^6$,
-C(=NH)$NH_2$ oder -NH-C(=NH)$NH_2$ substituiert sein kann,

$R^4$ Wasserstoff oder $C_1$-$C_6$-Alkyl, welches gegebenenfalls ein- oder zweifach durch einen oder zwei Reste ausgewählt aus der Gruppe Phenyl, Pyridyl und Naphthyl substituiert sein kann, wobei die Substituenten Phenyl und Naphthyl ihrerseits jeweils durch einen der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$, substituiert sein können;

$R^5$ und $R^6$ gleich oder verschieden, Wasserstoff, $C_1$-$C_4$-Alkyl, Pyridyl oder Benzyl, welches gegebenenfalls durch einen Rest ausgewählt aus der Gruppe -OH, -O-$C_1$-$C_3$-alkyl, -$NO_2$, Phenyl, Pyrrolidin-1-yl, -$NH_2$, -NH-$C_1$-$C_4$-alkyl, -N($C_1$-$C_4$-alykl)$_2$ und -C(=NH)$NH_2$ substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze:

Bevorzugt sind ferner Carboxamid-substituierte Benzimidazol-Derivate der allgemeinen Formel (I), worin

$R^1$ $C_1$-$C_4$-Alkyl, welches gegebenenfalls ein-, zwei- oder dreifach durch einen oder mehrere der Reste Hydroxy, $C_1$-$C_4$-Alkoxy, $CF_3$, Phenoxy, COOH, Halogen, -CO($C_1$-$C_4$-alkoxy), -CO-$NR^5R^6$, -$NR^5R^6$ oder $C_1$-$C_4$-Alkoxy-phenoxy substituiert sein kann, oder

$R^2$ -C(=NH)$NH_2$ oder -$CH_2$-$NH_2$;

$R^3$ ein $C_1$-$C_4$-Alkyl-Rest, der ein- oder zweifach durch einen oder zwei der Reste -$NR^5R^6$, -C(=NH)$NH_2$ oder -NH-C(=NH)$NH_2$ substituiert sein kann, oder ein direkt oder über eine Methylen- oder Ethylen-Brücke verknüpfter 5-, 6- oder 7-gliedriger, gesättigter oder ungesättigter Heterocyclus, der ein oder zwei Heteroatome ausgewählt aus der Gruppe Sauerstoff oder Stickstoff enthalten kann und der gegebenenfalls durch Methyl oder Benzyl substituiert sein kann;
Naphthylmethyl, Benzyl oder Phenylethyl, die an der Alkylenbrücke gegebenenfalls durch -$NR^5R^6$ substituiert sein können und die am Phenylring durch einen Rest ausgewählt aus der Gruppe -$NR^5R^6$, -$C_1$-$C_4$-Alkyl-$NR^5R^6$, -C(=NH)$NH_2$ und -NH-C(=NH)$NH_2$ substituiert sein können,

$R^4$ Wasserstoff oder $C_1$-$C_5$-Alkyl, welches gegebenenfalls ein- oder zweifach durch einen oder zwei Reste ausgewählt aus der Gruppe Pyridyl , Phenyl und Naphthyl substituiert sein kann;

$R^5$ und $R^6$ gleich oder verschieden, Wasserstoff, Methyl, Ethyl, Propyl, Butyl, Pyridyl oder Benzyl, welches gegebenenfalls durch einen Rest ausgewählt aus der Gruppe -OH, Methoxy, $-NO_2$, Phenyl, Pyrrolidin-1-yl, $-NH_2$, -NH-Methyl, $-N(Methyl)_2$, -NH-Ethyl, $-N(Ethyl)_2$ und $-C(=NH)NH_2$ substituiert sein kann, bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind Carboxamid-substituierte Benzimidazol-Derivate der allgemeinen Formel (I), worin

$R^1$ Methyl, Ethyl, Propyl oder Butyl, bevorzugt Methyl;

$R^2$ $-C(=NH)NH_2$ oder $-CH_2-NH_2$, bevorzugt $-C(=NH)NH_2$;

$R^3$ ein $C_2$-$C_4$-Alkyl-Rest, der ein- oder zweifach durch einen oder zwei der Reste $-NR^5R^6$, $-C(=NH)NH_2$ oder $-NH-C(=NH)NH_2$ substituiert sein kann, oder
ein über eine Methylen- oder Ethylen-Brücke verknüpfter 6-gliedriger, gesättigter oder ungesättigter Heterocyclus, der ein oder zwei Stickstoffatome enthält und der gegebenenfalls durch Methyl oder Benzyl substituiert sein kann;
Naphthylmethyl, Benzyl oder Phenylethyl, die an der Alkylenbrücke gegebenenfalls durch $-NR^5R^6$ substituiert sein können und die am Phenylring durch einen Rest ausgewählt aus der Gruppe $-NR^5R^6$, $-C_1$-$C_4$-Alkyl-$NR^5R^6$, $-C(=NH)NH_2$ und $-NH-C(=NH)NH_2$ substituiert sind,

$R^4$ Wasserstoff oder ein Alkylrest ausgewählt aus der Gruppe Methyl, Ethyl, Propyl, Butyl und Pentyl, der gegebenenfalls ein- oder zweifach durch einen oder zwei Reste ausgewählt aus der Gruppe Pyridyl , Phenyl und Naphthyl substituiert sein kann;

$R^5$ und $R^6$ gleich oder verschieden, Wasserstoff, Methyl, Ethyl, Propyl, Butyl, Pyridyl oder Benzyl, welches gegebenenfalls durch einen Rest ausgewählt aus der Gruppe -OH, Methoxy, $-NO_2$, Phenyl, Pyrrolidin-1-yl, $-NH_2$, -NH-Methyl, $-N(Methyl)_2$ und $-C(=NH)NH_2$ substituiert sein kann,

bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind femer Carboxamid-substituierte Benzimidazol-Derivate der allgemeinen Formel (I), worin

$R^1$ Methyl, Ethyl oder Propyl, bevorzugt Methyl;

$R^2$ $-C(=NH)NH_2$ oder $-CH_2-NH_2$, bevorzugt $-C(=NH)NH_2$;

$R^3$ Methyl, das durch einen Rest ausgewählt aus der Gruppe Pyridylamino, Benzylamino, N-Benzyl-N-methylamino, N-(Amidinobenzyl)amino, N-(Amidinobenzyl)-N-methyl-amino, N-(Dimethylaminobenzyl)amino, (Pyrrolidin-1-ylbenzyl)amino, und N-(Dimethylaminobenzyl)-N-methyl-amino substituiert ist, oder
ein Alkyl-Rest ausgewählt aus der Gruppe Ethyl und Propyl, der ein- oder zweifach durch einen oder zwei Reste ausgewählt aus der Gruppe $-NH_2$ und $-NH-C(=NH)NH_2$ substituiert sein kann, oder
ein über eine Ethylen-Brücke verknüpfter Heterocyclus ausgewählt aus der Gruppe Piperidin, Morpholin und Piperazin, der gegebenenfalls durch Methyl, Benzyl oder Diphenylmethyl substituiert sein kann;
Phenylethyl, das an der Ethylenbrücke gegebenenfalls durch $-NH_2$ substituiert sein kann und das am Phenylring durch einen Rest ausgewählt aus der Gruppe Pyrrolidin-1-yl, $-NH_2$, $-N(Methyl)_2$, $-CH_2-NH_2$ und $-C(=NH)NH_2$ substituiert ist;

$R^4$ Wasserstoff, Methyl, Ethyl, Propyl, Butyl, Pentyl, Benzyl, Pyridylmethyl , Naphthalinylmethyl oder Diphenylpropyl

bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Erfindungsgemäß von besonderer Bedeutung sind Carboxamid-substituierte Benzimidazol-Derivate der all-

gemeinen Formel (I), worin

$R^1$ Methyl, Ethyl oder Propyl, insbesondere Methyl;

$R^2$ $-C(=NH)NH_2$ oder $-CH_2-NH_2$, insbesondere $-C(=NH)NH_2$;

$R^3$ Methyl, das durch einen Rest ausgewählt aus der Gruppe N-(Amidinobenzyl)amino, N-(Amidinobenzyl)-N-methyl-amino, N-(Dimethylaminobenzyl)amino, (Pyrrolidin-1-ylbenzyl)amino, und N-(Dimethylaminobenzyl)-N-methyl-amino substituiert ist, oder
ein über eine Ethylen-Brücke verknüpftes Piperidin, das gegebenenfalls durch Benzyl substituiert sein kann;
Phenylethyl, das an der Ethylenbrücke gegebenenfalls durch $-NH_2$ substituiert sein kann und das am Phenylring durch $-C(=NH)NH_2$ substituiert ist;

$R^4$ Wasserstoff, Methyl, Butyl, Benzyl, Naphthalinylmethyl oder Diphenylpropyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind die Verbindungen der allgemeinen Formel (IA)

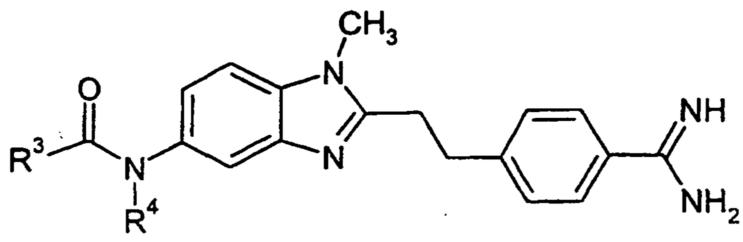
(IA),

worin

$R^3$ Methyl, das durch einen Rest ausgewählt aus der Gruppe N-(Amidinobenzyl)amino, N-(Amidinobenzyl)-N-methyl-amino, N-(Dimethylaminobenzyl)amino, (Pyrrolidin-1-ylbenzyl)amino, und N-(Dimethylaminobenzyl)-N-methyl-amino substituiert ist, oder
ein über eine Ethylen-Brücke verknüpftes Piperidin oder Piperazin, das gegebenenfalls durch Benzyl substituiert sein kann;
Phenylethyl, das an der Ethylenbrücke gegebenenfalls durch $-NH_2$ substituiert sein kann und das am Phenylring durch $-C(=NH)NH_2$ substituiert ist;

$R^4$ Wasserstoff, Methyl, Butyl, Benzyl, Naphthalinylmethyl oder Diphenylpropyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Neben den vorstehend genannten Verbindungen der allgemeinen Formel (I) zielt die vorliegende Erfindung femer auf Verbindungen, die aufgrund einer in vivo abspaltbaren Funktionalität erst nach ihrer Einnahme durch den Patienten vom Organismus in die therapeutisch wirksamen Verbindungen der allgemeinen Formel (I) überführt werden. Solche Verbindungen werden als Prodrugs bezeichnet. Ein weiterer Aspekt der vorliegenden Erfindung zielt dementsprechend auf Prodrugs der allgemeinen Formel (II)

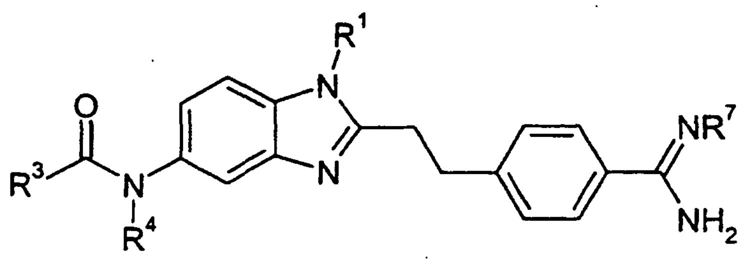

(II),

worin

$R^1$ und $R^4$ die vorstehend genannten Bedeutungen aufweisen können und

$R^3$ die vorstehend genannten Bedeutungen aufweisen kann oder $C_1$-$C_4$-Alkyl bedeutet, welches durch einen Rest ausgewählt aus der Gruppe
-C(=NOH)$NH_2$, -C(=NCOO-$C_1$-$C_{12}$-alkyl)$NH_2$ oder
-C(=NCOO-$C_1$-$C_8$-alkyl-Phenyl)$NH_2$ substituiert ist;

$R^7$ Hydroxy, -COO-$C_1$-$C_{12}$-Alkyl, -CO-Phenyl, -CO-Pyridyl oder -COO-$C_1$-$C_8$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann,

mit der Maßgabe, dass Verbindungen der Formel 1, worin

$R^3$ eine $C_1$-$C_5$-Alkyl-, Phenyl-$C_1$-$C_3$-alkyl-, $C_3$-$C_6$-Cycloalkyl-, Pyridylgruppe, und
$R^4$ ein Wasserstoffatom, $C_1$-$C_5$ Alkyl oder Phenyl-$C_1$-$C_3$-alkylgruppe

bedeuten, ausgenommen sind, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Bevorzugt sind Prodrugs der allgemeinen Formel (II), worin

$R^1$ und $R^4$ die vorstehend genannten Bedeutungen aufweisen können und

$R^3$ die vorstehend genannten Bedeutungen aufweisen kann oder $C_1$-$C_4$-Alkyl bedeutet, welches durch einen Rest ausgewählt aus der Gruppe
-C(=NOH)$NH_2$, -C(=NCOO-$C_1$-$C_6$-alkyl)$NH_2$ oder
-C(=NCOO-$C_1$-$C_6$-alkyl-Phenyl)$NH_2$ substituiert ist;

$R^7$ Hydroxy, -COO-$C_1$-$C_6$-Alkyl, -CO-Phenyl, -CO-Pyridyl oder -COO-$C_1$-$C_6$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Besonders bevorzugt sind Prodrugs der allgemeinen Formel (II), worin

$R^1$, $R^3$ und $R^4$ die vorstehend genannten Bedeutungen aufweisen können und

$R^7$ Hydroxy, Methoxycarbonyl, Ethoxycarbonyl, Propyloxycarbonyl, Butyloxycarbonyl, Benzoyl, Benzyloxycarbonyl oder Nicotinoyl bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Als Alkylgruppen (auch soweit sie Bestandteil anderer Reste sind) werden, falls nicht anders definiert, verzweigte und unverzweigte Alkylgruppen mit 1 bis 12 Kohlenstoffatomen, bevorzugt 1 bis 8 Kohelnstoffatomen, besonders bevorzugt mit 1 bis 6 Kohelnstoffatomen betrachtet. Beispielsweise werden genannt: Methyl, Ethyl, Propyl, Butyl, Pentyl, Hexyl etc. Sofern nicht anders genannt, sind von den vorstehend genannten Bezeichnungen Propyl, Butyl, Pentyl oder Hexyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc. Gegebenfalls werden zur Bezeichnung der vorstehend genannten Alkylreste auch gängige Abkürzungen wie Me für Methyl, Et für Ethyl etc. verwendet.

Als Alkenylgruppen (auch soweit sie Bestandteil anderer Reste sind) werden verzweigte und unverzweigte Alkenylgruppen mit 2 bis 6 Kohlenstoffatomen, bevorzugt 2 bis 4 Kohlenstoffatomen genannt, soweit sie mindestens eine Doppelbindung aufweisen, beispielsweise auch oben genannte Alkylgruppen bezeichnet, soweit sie mindestens eine Doppelbindung aufweisen, wie zum Beispiel Vinyl (soweit keine unbeständigen Enamine oder Enolether gebildet werden), Propenyl, iso-Propenyl, Butenyl, Pentenyl, Hexenyl.

Als Alkinylgruppen (auch soweit sie Bestandteil anderer Reste sind) werden Alkinylgrupppen mit 2 bis 6 Kohlenstoffatomen bezeichnet, soweit sie mindestens eine Dreifachbindung aufweisen, beispielsweise Ethinyl, Propargyl, Butinyl, Pentinyl, Hexinyl.

Als Halogen wird im allgemeinen Fluor, Chlor, Brom oder Jod bezeichnet.

Als 5- ,6- oder 7-gliedrige, gesättigte oder ungesättigte Heterocyclen, die als Heteroatome Stickstoff, Sauerstoff oder Schwefel enthalten können, werden, soweit in den Definitionen nicht anders beschrieben beispielsweise Furan, Tetrahydrofuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Pyrazolidin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Diazepan, Oxazol, Isoxazol, Oxazin, Thiazol, Isothiazol, Thiadiazol, Oxadiazol, Pyrazolidin genannt, wobei der Heterocyclus wie in den Definitionen angegeben substituiert sein kann.

"=O" bedeutet ein über eine Doppelbindung verknüpftes Sauerstoffatom.

Die vorliegende Erfindung zielt ferner auf die Verwendung der vorstehend definierten Verbindungen der allgemeinen Formel (I) sowie auf die der Prodrugs der allgemeinen Formel (II) zur Herstellung eines Arzneimittels zur Behandlung von Krankheiten, in denen Tryptase-Inhibitoren einen therapeutischen Nutzen entfalten können.

Erfindungsgemäß bevorzugt ist die vorstehend genannte Verwendung von Verbindungen der allgemeinen Formel (I) zur Herstellung eines Arzneimittels zur Vorbeugung und/oder Behandlung entzündlicher und/oder allergischer Erkrankungen. Besonders bevorzugt ist die eingangs genannte Verwendung der Verbindungen der allgemeinen Formel (I) zur Herstellung eines Arzneimittels zur Vorbeugung und/oder Behandlung von Asthma bronchiale, allergischer Rhinitis, allergischer Conjunctivitis, atopischer Dermatitis, Urticaria, allergischer Otitis, allergischer Magen-Darmerkrankungen, Morbus Crohn, Colitis ulcerosa, anaphylaktischer Schock, septischer Schock, Schocklunge (ARDS) und Arthritis. Ferner ist von Interesse die eingangs genannte Verwendung der Verbindungen der allgemeinen Formel (I) zur Herstellung eines Arzneimittels zur Vorbeugung und/oder Behandlung von Fibrosen wie Lungenfibrose, fibrosierende Alveolitis und Narbenbildung, von Kollagenosen wie Lupus erythematodes und Sklerodermie sowie von Arteriosklerose, Psoriasis und Neoplasien.

Die Synthese der substituierten Benzimidazol-Derivate der Formel (I) sowie die der Prodrugs der allgemeinen Formel (II) kann über unterschiedliche synthetische Zugänge erfolgen. Mögliche Zugänge in Anlehnung an und unter Verwendung von konventionellen chemischen Synthesemethoden werden im Folgenden exemplarisch dargestellt. Aus Schema 1 ist eine mögliche Vorgehensweise zum Aufbau des Benzimidazolgrundkörpers der erfindungesgemäßen Verbindungen ersichtlich.

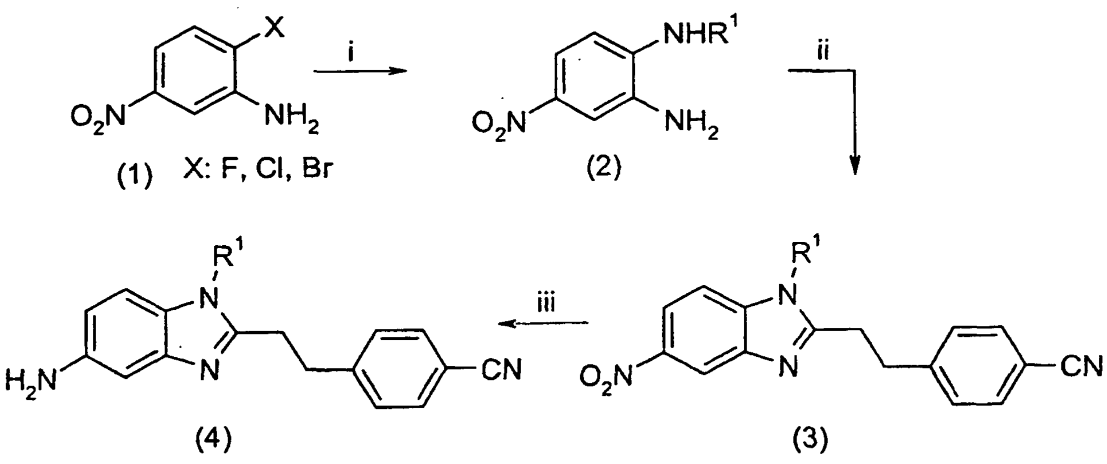


(1) X: F, Cl, Br

(2)

(4)

(3)

Schema 1:

Ausgehend von den 2-Halogen-5-nitro-anilinen (1) kann zunächst eine Aminolyse zu den Diaminonitrobenzolen (2) gemäß Schema 1 (Stufe i) erfolgen. Die Aminolyse der Verbindungen (1) mit den primären Aminen $R^1$-$NH_2$ erfolgt in geeigneten organischen Lösemitteln wie beispielsweise Dimethylsulfoxid, N,N-Dimethylformamid, N-Methylpyrrolidon, Aceton oder gegebenenfalls auch in Wasser oder Alkoholen bei Raumtemperetur oder in einem Temperaturbereich von 30-80°C, bevorzugt 40-50°C.

Die Umsetzung der Verbindungen (2) mit p-Cyanophenylpropionsäure führt zu den Nitro-benzimidazolen (3, Stufe ii) durch Umsetzung mit p-Cyanophenylpropionsäure in Gegenwart dehydratisierender Reagentien. Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan durchgeführt. Als dehydratisierende Mittel kommen beispielsweise in Betracht Chlorameisensäureisobutylester, Orthokohlensäuretetraethylester, Orthoessigsäuretrimethylester, 2,2-Dimethoxypropan, Tetramethoxysilan, Phosphoroxychlorid, Thionylchlorid, Trimethylchlorsilan, Phosphortrichlorid, Phosphorpentoxid, 1,2-Dihydro-2-ethoxy-chinolin-1-carbonsäureethylester (EEDQ), 1,2-Dihydro-2-*i*-propyloxy-chinolin-1-carbonsäure-*i*-propylester (IIDQ), N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1 H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff. Gegebenenfalls kann sich der Zusatz einer Base wie Pyridin, 4-Dimethylaminopyridin, N-Methyl-morpholin oder Triethylamin als zweckmäßig erweisen. Die Umsetzung erfolgt üblicherweise bei Temperaturen zwischen 0 und 150°C, vorzugsweise bei Temperaturen zwischen 20 und 120°C.

Die gemäß vorstehend beschriebener Vorgehensweise erhältlichen Nitrobenzimidazol-Derivate (3) können reduktiv in die Amino-benzimidazole (4) überführt werden (Stufe iii, Schema 1). Die Reduktion der Nitro-gruppe zu den Verbindungen (3) gelingt beispielsweise durch katalytische Hydrierungen in organischen Lösemitteln wie beispielsweise Methanol, Ethanol, Isopropanol, Tetrahydrofuran, gegebenenfalls auch im Gemisch mit Dimethylformamid, Ethylacetat, Dioxan oder Essigsäure, bei erhöhtem Wasserstoffdruck oder bei Normaldruck bei Temperaturen zwischen 0-50°C, vorzugsweise bei 20-40°C. Als Katalysatoren kommen gängige Hydrierkatalysatoren in Betracht. Bevorzugt sind Palladium und Raney-Nickel. Erfindungsgemäß kommt vorzugsweise Palladium in Betracht. Besonders bevorzugt ist als Katalysator Palladium auf Kohle (5%). Eine alternative Vorgehensweise zur Reduktion der Nitroverbindungen (3) sieht die Verwendung von Reduktionsmitteln wie $Na_2S_2O_4$ oder $SnCl_2$ vor. Diese Umsetzung erfolgt in protischen, mit Wasser mischbaren organischen Lösemitteln wie kurzkettigen Alkoholen (Methanol, Ethanol, Isopropanol) oder in einer Mischung vorstehend genannter Lösemittel mit Wasser, gegebenenfalls mit Essigsäure, Dimethylformamid oder Ethylacetat. Die Reaktion wird üblicherweise bei erhöhter Temperatur, vorzugsweise unter Rückfluß des jeweiligen Lösemittels oder Lösemittelgemischs geführt. Nach vollständigem Umsatz der Ausgangsverbindungen (3) wird auf üblichem Wege aufgearbeitet. Eine Reinigung der Verbindungen (4) kann beispielsweise durch Kristallisation aus unpolareren organischen Lösemitteln wei Diethylether, Petrolether, gegebenenfalls im Gemisch mit Ethylacetat erfolgen.

Ausgehend von den gemäß Schema 1 erhältlichen Benzimidazolen (4) erfolgt die Bildung der Verbindungen (5) gemäß Schema 2 durch Umsetzung mit den Verbindungen $R^4$-Nu, wobei Nu für eine nukleofuge Austrittsgruppe wie beispielsweise Chlor, Brom, Iod, Methansulfonat, Methyltriflat, p-Toluolsulfonat etc. steht. Alternativ dazu können

die Verbindungen (5) ausgehend von den Verbindungen (4) auch im Sinne einer reduktiven Aminierung durch Umsetzung mit entsprechend substituierten Ketonen oder Aldehyden unter reduktiven Bedingungen erhalten werden.

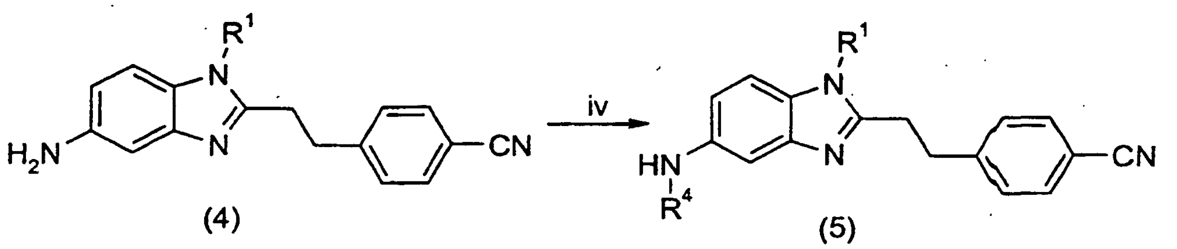


(4) (5)

Schema 2:

Zur Umsetzung der Verbindungen (4) mit $R^4$-Nu gemäß Stufe iv kann wie folgt vorgegangen werden. Eine Verbindung (4) wird in einem polaren Lösungsmittels, wie Dimethylformamid, Dimethylactamid, Methylenchlorid, Tetrahydrofuran, bevorzugt Dimethylformamid und besonders bevorzugt wasserfreies, gegebenenfalls absolutes Dimethylformamid gelöst. Die so erhaltene Lösung wird mit einer Base und dem entsprechenden Alkylierungsmittel R4-Nu versetzt. Als Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht. Ferner sind die Alkali- oder Erdalkalihydroxide des Lithiums, Natriums, Kaliums, Magnesiums, Calziums, bevorzugt jedoch Natriumhydroxid, Kaliumhydroxid, Lithiumhydroxid und Calziumhydroxid in Alkoholen oder Wasser einsetzbar. Das Reaktionsgemisch wird 0.5-8h, bevorzugt 1-4h bei erhöhter Temperatur, vorzugsweise bei 50-120°C, besonders unter Rückfluß des verwendeten Lösemittels gerührt. Nach vollständigem Umsatz wird auf üblichem Wege aufgearbeitet und das erhaltene Rohprodukt durch Kristallisation oder Chromatographie an Kieselgel gereinigt.
Werden die Verbindungen (5) aus den Verbindungen (4) durch reduktive Aminierung erhalten, wird wie folgt vorgegangen. In einem geeigneten Lösemittel wie beispielsweise Dichlormethan, Dichlorethan, Methanol, Ethanol, Tetrahydrofuran oder Toluol wird die Verbindung (4) gelöst und zwischen 0-60°C, vorzugsweise bei 20-40°C mit der entsprechenden Carbonylverbindung in Gegenwart einer Säure, vorzugsweise einer Carbonsäure, besonders bevorzugt einer kurzkettigen Carbonsäure, höchst bevorzugt Essigsäure versetzt. Anschließend erfolgt die Zugabe eines geeigneten Reduktionsmittels. Als Reduktionsmittel kommen erfindungsgemäß in Betracht $Na[HB(OAc)_3]$, $Na[BH_3CN]$, $NaBH_4$, Pd/C-$H_2$, bevorzugt ist $Na[HB(OAc)_3]$. Nach üblicher Aufarbeitung erfolgt die Reinigung des Produkts durch Kristallisation oder Chromatographie an Kieselgel.

Aus den Verbindungen (5) lassen sich, wie aus Schema 3 ersichtlich, durch Acylierung die Intermediate der allgemeinen Formel (III) erhalten (Stufe v).

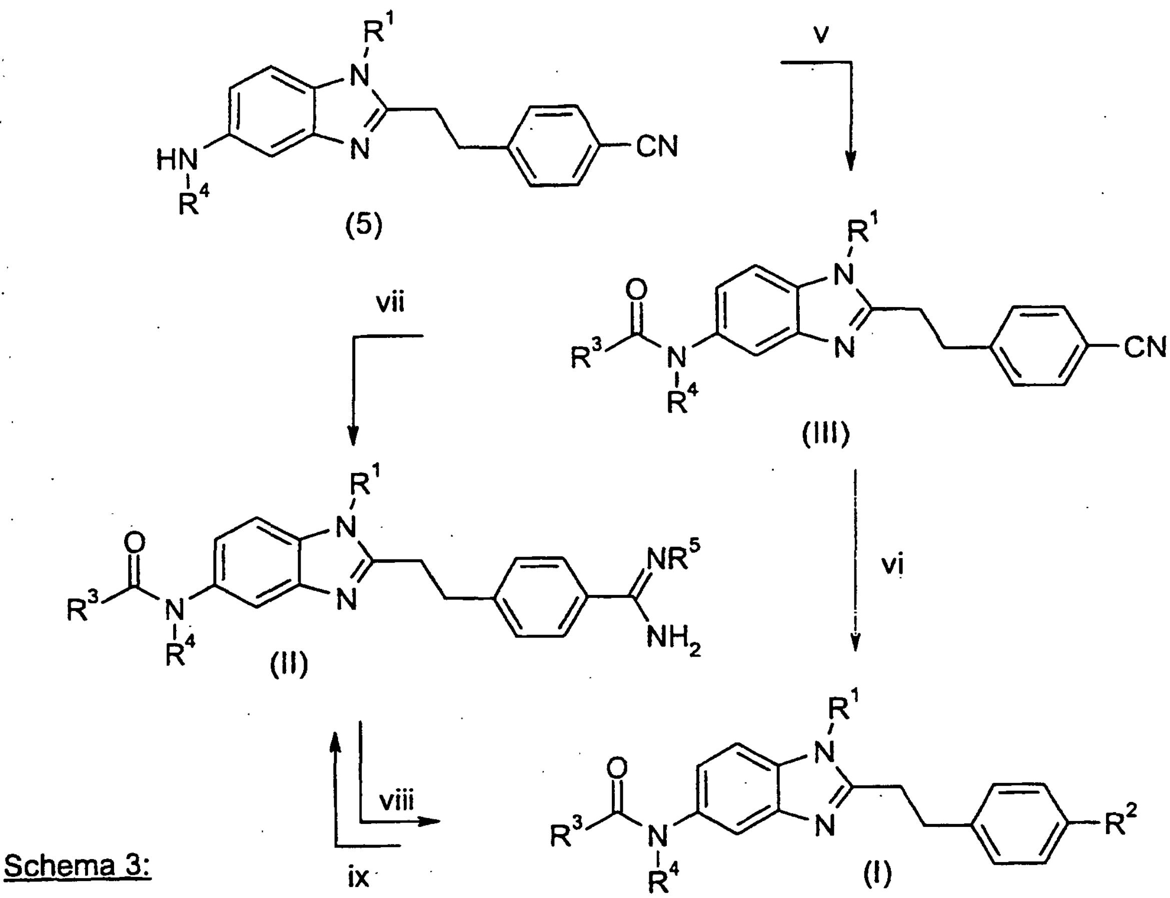

(5)
(III)
(II)
(I)

Schema 3:

Die Umsetzung der Verbindungen (5) mit den Carbonsäuren $R^3$-COOH zu den intermediaten der allgemeinen Formel (III) kann in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt werden.
Altemativ dazu können die Intermediate der allgemeinen Formel (III) auch über Standardverfahren durch Umsetzung mit entsprechend aktivierten Carbonsäurederivaten $R^3$-COX (mit X: Halogenid, Alkoxy, etc.) in den vorstehend genannten Lösemitteln bzw. Lösemittelgemischen in Gegenwart von Basen wie Pyridin, 4-Dimethylaminopyridin, N-Methyl-morpholin oder Triethylamin erhalten werden.

Gemäß Stufe vi sind aus den Intermediaten (III) die erfindungsgemäßen Verbindungen der allgmeinen Formel (I) zugänglich. Zur Herstellung der erfindungsgemäßen Verbindungen der allgemeinen Formel (I) in denen $R^2$-C(=NH)$NH_2$ bedeutet, kann auf unterscheidliche Art und Weise vorgegangen werden.
Eine Verbindung der allgemeinen Formel (I) erhält man beispielsweise durch Behandlung einer Verbindung der allgemeinen Formel (III) mit einem entsprechenden Alkohol wie Methanol, Ethanol, n-Propanol, Isopropanol oder Benzylalkohol gegebenenfalls im Gemisch mit einem anderen organischen Lösungsmittel wie beispielsweise Chloroform, Nitrobenzol oder Toluol in Gegenwart einer Säure wie Salzsäure oder durch Umsetzung eines entsprechenden Amids mit einem Trialkyloxoniumsalz wie Triethyloxonium-tetrafluorborat in einem Lösemittel wie Methylenchlorid, Tetrahydrofuran oder Dioxan bei Temperaturen zwischen -10 und 50°C, vorzugsweise jedoch bei 0-20°C und nachfolgender Aminolyse mit beispielsweise alkoholischer Ammoniaklösung. Altemativ dazu lassen sich die Verbindungen der allgemeinen Formel (I) erhalten durch Umsetzung einer Verbindung der allgemeinen Formel (III) mit Schwefelnukleophilen wie z.B. Schwefelwasserstoff, Ammonium- bzw. Natriumsulfid, Natriumhydrogensulfid, Kohlenstoffdisulfid, Thioacetamid oder Bistrimethylsilylthioether gegebenenfalls in Gegenwart von Basen wie Triethylamin, Ammoniak, Natriumhydrid oder Natriumalkoholat in Lösungsmitteln wie Methanol, Ethanol, Wasser, Tetrahydrofuran, Pyridin , Dimethylformamid oder 1,3-Dimethyl-imidazolidin-2-on bei 20-100 °C und anschließende Behandlung mit einem geeigneten Methylierungsmittel wie z.B. Methyliodid oder Dimethylsulfat in einem Lösungsmittel wie Acetonitril oder Aceton bei Temperaturen zwischen -10 und 50°C, vorzugsweise jedoch bei 0-20°C und anschließende Behandlung mit Ammoniak, Ammoniumcarbonat oder Ammoniumchlorid in einem geeigneten Alkohol, wie beispielsweise Methanol, Ethanol, Isopropanol etc. bei Temperaturen zwischen
-10 und 50°C, vorzugsweise jedoch bei 0-20°C.

Ferner sind die erfindungsgemäßen Verbindungen der allgemeinen Formel (I) zugänglich durch Behandlung einer Verbindung der allgemeinen Formel (III) mit Lithiumhexamethyldisilazid in einem geeigneten organischen Lösungsmittel wie z.B. Tetrahydrofuran bei Temperaturen zwischen -20 und 50 °C, vorzugsweise jedoch bei 0-20 °C und anschließende Hydrolyse mit verdünnter Salzsäure bei 0-5 °C.

Ein weiterer altemativer Zugang zu Verbindungen der allgemeinen Formel (I) gelingt durch Behandlung einer Verbindung der allgemeinen Formel (III) mit Ammoniumchlorid und Trimethylaluminium in einem geeigneten organischen Lösungsmittel wie z.B. Toluol bei Temperaturen zwischen 20 und 150 °C, vorzugsweise jedoch bei 110°C.

Verbindungen der allgemeinen Formel (I) in denen $R^2$-$CH_2$-$NH_2$ bedeutet, lassen sich aus den Intermediaten (III) beispielsweise durch katalytische Hydrierung an Raney-Nickel erhalten. Diese Umsetzungen werden vorzugsweise in protischen organischen Lösemitteln wie kurzkettigen Alkoholen (Methanol, Ethanol oder Isopropanol) bei Temperaturen zwischen 10-40°C, vorzugsweise bei 20-30°C unter Normaldruck durchgeführt.

Eine Verbindung der allgemeinen Formel (II) erhält man beispielsweise durch Behandlung einer Verbindung der allgemeinen Formel (III, Schema 3, Stufe vii) mit Hydroxylamin in Gegenwart von Carbonaten oder Alkoholaten der Alkali- oder Erdalkalimetalle in Lösemitteln wie Methanol, Ethanol, n-Propanol oder Isopropanol gegebenenfalls im Gemisch mit Dioxan oder Tetrahydrofuran. Die Alkoholate können dargestellt werden aus den jeweiligen Alkalimetallen oder Metallhydriden und dem entsprechenden Alkohol. Die Reaktion wird vorzugsweise bei 20-100°C, besonders bevorzugt bei der Siedetemperatur des verwendeten Lösemittels durchgeführt. Verbindungen der allgemeinen Formel (II) sind alternativ zugänglich durch Behandlung einer Verbindung der allgemeinen Formel (III) mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösemittel wie Methylenchlorid, Tetrahydrofuran oder Dioxan bei Temperaturen zwischen -10 und 50°C, vorzugsweise jedoch bei 0-20°C und anschließende Behandlung mit Hydroxylamin in Gegenwart von Basen in einem geeigneten Alkohol, wie Methanol, Ethanol, Isopropanol etc. bei Temperaturen zwischen -10 und 50°C, vorzugsweise jedoch bei 0-20°C.

Eine Verbindung der allgemeinen Formel (I) erhält man beispielsweise durch Behandlung einer Verbindung der allgemeinen Formel (II, Schema 3, Stufe viii) mit Wasserstoff in Gegenwart von Hydrierkatalysatoren wie Raney-Nikkel oder Rhodium/Aluminiumoxid in Wasser oder Methanol gegebenenfalls unter Zusatz von Säuren wie Salzsäure oder Methansulfonsäure oder durch Behandlung mit Wasserstoff in Gegenwart von Palladium/Kohle in Essigsäure/Essigsäureanhydrid bei 20-50 °C und 1-5 bar Wasserstoffdruck, bevorzugt bei Raumtemperatur und Normaldruck.

Die Acyl- oder Alkoxycarbonyl-Prodrugs (II) der Verbindung mit der allgemeinen Formel (I) erhält man durch Umsetzung der Verbindungen der allgemeinen Formel (I) mit den entsprechenden Säurechloriden in Gegenwart von Basen wie z.B. Triethylamin, N-Methylmorpholin, Diethylisopropylamin oder DBU in einem geeigneten Lösungsmittel wie Methylenchlorid, Chloroform, Tetrahydrofuran, Acetonitril, Dimethylformamid oder Dimethylsulfoxid.

Ihrer zentralen Bedeutung für die Synthese der erfindungsgemäßen Verbindungen der allgemeinen Formel (I) sowie für die Synthese der Prodrugs der allgemeinen Formel (II) entsprechend, zielt ein weiterer Aspekt der vorliegenden Erfindung auf die Intermediate der allgemeinen Formel (III)

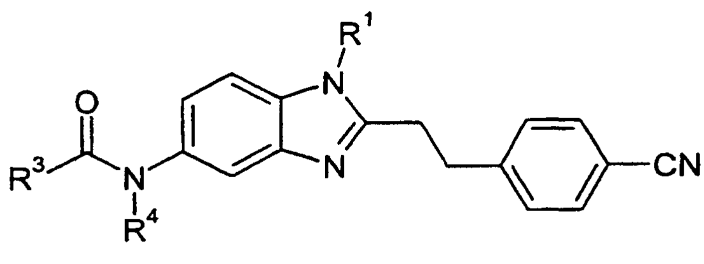

(III)

in der die Reste $R^1$, $R^3$ und $R^4$ die vorstehende Bedeutung aufweisen können.

Die Verbindungen der allgemeinen Formel (III), stellen, wertvolle Zwischenprodukte zur Herstellung der erfindungsgemäßen Benzimidazol-Derivate der allgemeinen Formel (I) sowie der der erfindungsgemäßen Prodrugs der allgemeinen Formel (II) dar.

Aufgrund ihrer pharmakologischen Eigenschaften können die erfindungsgemäßen Verbindungen als Arzneimittel, insbesondere als Arzneimittel mit Tryptase-inhibierender Wirkung Verwendung finden. Sind überall dort anwendbar, wo Tryptase-Inhibitoren einen therapeutischen Nutzen entfalten können. Erfindungsgemäß bevorzugt ist die Verwendung von Verbindungen der allgemeinen Formel (I) zur Herstellung eines Arzneimittels zur Vorbeugung und/oder Behandlung entzündlicher und/oder allergischer Erkrankungen. Besonders bevorzugt ist die eingangs genannte Verwendung der Verbindungen der allgemeinen Formel (I) zur Herstellung eines Arzneimittels zur Vorbeugung und/oder Behandlung von Asthma bronchiale, allergischer Rhinitis, allergischer Conjunctivitis, atopischer Dermatitis, Urticaria, allergischer Otitis, allergischer Magen-Darmerkrankungen, Morbus Crohn, Colitis ulcerosa, anaphylaktischer Schock, septischer Schock, Schocklunge (ARDS) und Arthritis.

Femer ist von Interesse die eingangs genannte Verwendung der Verbindungen der allgemeinen Formel (I) zur Herstellung eines Arzneimittels zur Vorbeugung und/oder Behandlung von Fibrosen wie Lungenfibrose, fibrosierende Alveolitis und Narbenbildung, von Kollagenosen wie Lupus erythematodes und Sklerodermie sowie von Arteriosklerose, Psoriasis und Neoplasien.

Im Folgenden werden exemplarische Vorgehensweisen zur Herstellung der erfindungsgemäßen Verbindungen detaillierter beschrieben. Die nachfolgenden Synthesebeispiele dienen ausschließlich einer detaillierteren Erläuterung.

**Beispiel 1: N-{2-[2-(4-Amidinophenyl)-ethyl]-1-methyl-benzimidazol-5-yl}-N-methyl-[3-(4-amidinophenyl)-propionamid] dihydrochlorid**

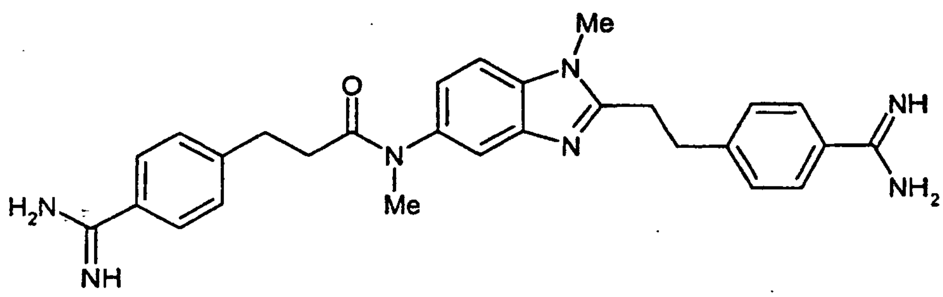

a) $N^1$-Methyl-1,2-diamino-4-nitrobenzol

2-Fluor-5-nitro-anilin (15,0 g, 160 mmol) wird in 480 mL 40%iger wäßriger Methylaminlösung aufgenommen, 2,5 Tage bei Raumtemperatur und 2 h bei 40-50 °C gerührt. Es wird mit Wasser verdünnt, der Feststoff abfiltriert, mit Wasser gewaschen und getrocknet.
Ausbeute: 26 g (97%); Schmp.: 171-173 °C.

b) 2-[2-(4-Cyanophenyl)-ethyl]-1-methyl-5-nitro-benzimidazol

$N^1$-Methyl-1,2-diamino-4-nitrobenzol (8,3 g, 49,6 mmol) und *p*-Cyanophenylpropionsäure (9,6 g, 55 mmol) werden in 90 mL $POCl_3$ aufgenommen, und 1,5 h unter Rückfluß erhitzt. Nach dem Abkühlen wird das überschüssige $POCl_3$ mit Eiswasser zersetzt. Es wird mit $NH_3$ unter Rühren / Kühlung alkalisch gestellt und 1 h bei Raumtemperatur gerührt. Der Feststoff wird abfiltriert, mit Wasser gewaschen und aus DMF umkristallisiert.
Ausbeute: 11,7 g (76,4%); Schmp.: 202-204 °C.

c) 5-Amino-2-[2-(4-cyanophenyl)-ethyl]-1-methyl-benzimidazol

2-[2-(4-Cyanophenyl)-ethyl]-1-methyl-5-nitro-benzimidazol (5,5 g, 18 mmol) in 150 mL THF/75 mL Methanol wird in Gegenwart von 1,0 g 5% Pd/C bei Raumtemperatur und Normaldruck hydriert. Der Katalysator wird abfiltriert, das Filtrat nicht komplett zur Trockene eingeengt, mit 100 mL Acetonitril verdünnt und bis zu einem Restvolumen von 30 mL eingeengt. Der Kristallbrei wird abgekühlt und filtriert. Die Kristalle werden mit kaltem Acetonitril und Ether gewaschen.
Ausbeute: 4,7 g (94%); Schmp.: 187-192 °C.

d) N-{2-[2-(4-Cyanophenyl)-ethyl]-1-methyl-benzimidazol-5-yl}-trifluoracetamid

4.1 g (15 mmol) 5-Amino-2-[2-(4-cyanophenyl)-ethyl]-1-methyl-benzimidazol werden in 40 mL Pyridin bei 5-20°C tropfenweise mit Trifluoressigsäureanhydrid (2.2 mL) versetzt. Es wird 15 min. bei Raumtemperatur gerührt und mit Wasser verdünnt. Der Feststoff wird abfiltriert und mit Wasser gewaschen. Die Kristalle werden unter Erwärmen in 300 mL Ethylacetat gelöst, getrocknet und nicht ganz zur Trockene eingeengt. Es wird abgekühlt, der ausgefallene Feststoff abfiltriert und mit Diethylether gewaschen.
Ausbeute: 4.1g (74%); Schmp.: 225-228 °C.

e) 2-[2-(4-Cyanophenyl)-ethyl]-1-methyl-5-methylamino-benzimidazol

4.1g (11 mmol) N-{2-[2-(4-Cyanophenyl)-ethyl]-1-methyl-benzimidazol-5-yl}-trifluoracetamid werden in 20 mL Dimethylsulfoxid auf ca. 10 °C abgekühlt und unter Rühren portionweise mit 80% igem NaH (0.33 g, ca. 11 mmol) versetzt. Es wird für 0.5 h auf 40 - 50 °C erwärmt und anschließend auf 30 °C abgekühlt. Im Anschluß wird Methyliodid (0.75 mL, 11.9 mmol) zugetropft. Es wird 0.5h bei 40-50°C gerührt, mit 100 mL Ethylacetat verdünnt, auf Wasser gegossen und extrahiert. Die organische Phase wird nochmals mit Wasser gewaschen, gertrocknet und eingeengt. Der Rückstand wird über Kieselgel chromatographiert. Das methylierte Trifluoracetamid wird in MeOH aufgenommen, mit 10 mL konz. wässriger Ammoniaklösung versetzt und 4 h bei 30-40 °C gerührt. Das Methanol wird abdestilliert, der Rückstand in 75 mL Ethylacetat aufgenommen, mit Wasser gewaschen, getrocknet und eingeengt. Das Produkt wird aus Diethylether kristallisiert. Ausbeute: 2.0 g (63 %); Schmp.: 155-158 °C.

f) N-{2-[2-(4-Cyanophenyl)-ethyl]-1-methyl-benzimidazol-5-yl}-N-methyl-[3-(4-cyanophenyl)-propionamid]

2-[2-(4-Cyanophenyl)-ethyl]-1-methyl-5-methylamino-benzimidazol (0.9 g, 3.1 mmol), 3-(*p*-Cyanophenyl)-propionsäure (0.6 g, 3,4 mmol) und 0.5 mL N-Methylmorpholin werden in 10 mL Dimethylfomamid aufgenommen. Es wird TBTU (1.2 g, 3.7 mmol) zugesetzt und 16 h bei Raumtemperatur gerührt. Nach dem Verdünnen mit 75 mL Ethylacetat wird mit verd., wässriger NaOH- bzw. ges. $NaHCO_3$-Lsg. und mit Wasser gewaschen, getrocknet und eingeengt. Das Produkt wird aus Ethylacetat/Diethylether kristallisiert.
Ausbeute: 1.3 g (94 %); Schmp.: 138-140 °C.

g) N-{2-[2-(4-Amidinophenyl)-ethyl]-1-methyl-benzimidazol-5-yl}-N-methyl-[3-(4-amidinophenyl)-propionamid]-dihydrochlorid

N-{2-[2-(4-Cyanophenyl)-ethyl]-1-methyl-benzimidazol-5-yl}-N-methyl-[3-(4-cyanophenyl)-propionamid] (1.3 g, 2.9 mmol) wird in 50 mL einer gekühlten bei 0 °C gesättigten ethanolischen HCl-Lösung aufgenommen. Es wird bis zur vollständigen Auflösung des Eduktes gerührt und anschließend über Nacht bei 0-5 °C gehalten. Das Ethanol wird bei maximal 40 °C abdestilliert und der Rückstand in 40 mL einer bei 0 °C gesättigten ethanolischen Ammoniak-Lösung aufgenommen. Es wird 2 h bei Raumtemperatur, 3 h bei 50-60 ° gerührt, mit weiteren 10 mL ges. Ammoniak-Lösung versetzt, 2 h unter Rückfluß gekocht und über Nacht bei Raumtemperatur gehalten. Die ausgefallenen anorganischen Salze werden abfiltriert, das Filtrat eingeengt und der Rückstand über Kieselgel chromatographiert.
Ausbeute: 1.0 g (62%);
Masse: ber.: [481], gef.: $[M+H]^+$ 482.
$^1$H-NMR (250MHz,$CD_3OD$):δ=7.86-7.06 (11H,m,aryl-H); 3.81 (3H,s,N-$CH_3$); 3.32 (4H;s;-$CH_2$-$CH_2$-); 3.29 (3H,s, O=C-N-$CH_3$); 3.02; 2.46 (4H, 2t,J=7.6Hz, O=C-$CH_2CH_2$-).

**Beispiel 2: N-{2-[2-(4-Amidinophenyl)-ethyl]-1-methyl-benzimidazol-5-yl}-N-methyl-[3-(N-benzyl-piperid-4-yl)-propionamid]-dihydrochlorid**

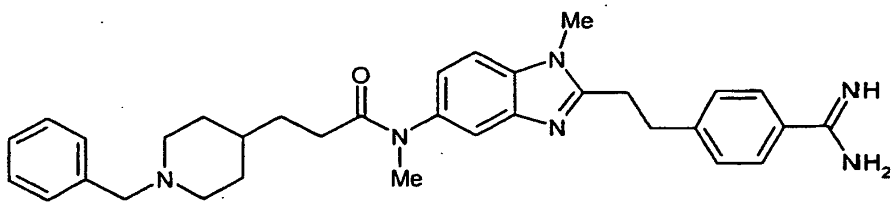

Die Synthese gelingt ausgehend von gemäß Beispiel 1, Stufe e erhältlichem 2-[2-(4-Cyanophenyl)-ethyl]-1-methyl-5-methylamino-benzimidazol (2.4 mmol), und 3-(N-benzyl-piperid-4-yl)-propionsäure durch Kupplung mit TBTU analog zu der in Beispiel 1, Stufe f beschriebenen Vorgehensweise. Die Reinigung der gemäß Beispiel 1, Stufe g erhältlichen Titelverbindung erfolgt durch Chromatographie an Kieselgel.
Ausbeute: 61 %;
Masse: : ber.: [536], gef.: $[M+H]^+$ 537, $[M+2H]^{2+}$ 269.
$^1$H-NMR (250MHz, $CD_3OD$):δ=7.98-7.34 (12H, m, aryl-H); 4.20 (2H, s, N-$CH_2$); 3.97 (3H, s, N-$CH_3$); 3.61; 3.36 (4H, 2m,-$CH_2$-$CH_2$-); 3.05-1.20 (13H, m, Piperidinylethyl); 3.32 (3H, s, O=C-N-$CH_3$).

<u>Beispiel 3:</u> **N-{2-[2-(4-Amidinophenyl)-ethyl]-1-benzyl-benzimidazol-5-yl}-N-methyl-[3-(N-benzyl-piperid-4-yl)-propionamid]-dihydrochlorid**

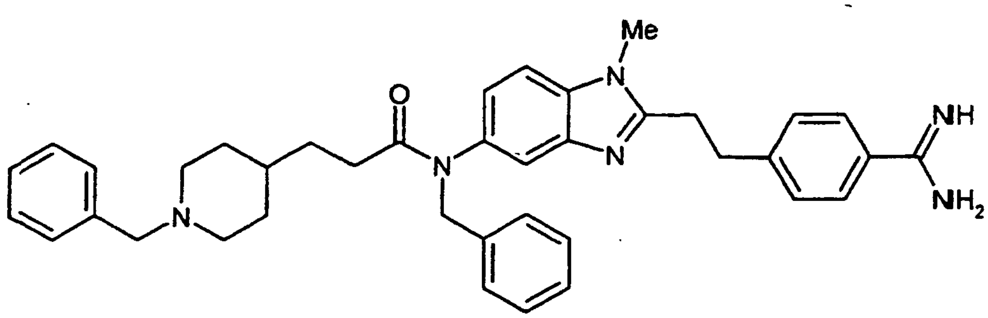

Die Synthese gelingt in Analogie zu der für Beispiel 2 beschriebenen Vorgehensweise. Die Reinigung der Titelverbindung erfolgt durch Chromatographie an Kieselgel. Ausbeute: 53%.
Masse: ber.: [612], gef.: $[M+H]^+$ 613, $[M+2H]^{2+}$ 307.
$^1$H-NMR (250MHz, $CD_3OD$):δ=7.97-7.21 (17H, m, aryl-H); 5.04; 4.32 (4H, 2s, N-$CH_2$-Ph); 3.94 (3H, s, N-$CH_3$); 3.44; 2.97 (4H, 2m, -$CH_2$-$CH_2$); 3.48-1.22 (13H, m, Piperidinylethyl).

<u>Beispiel 4:</u> **N-{2-[2-(4-Amidinophenyl)-ethyl]-1-methyl-benzimidazol-5-yl}-[2-amino-3-(*p*-amidinophenyl)-propionsäureamid]-trihydrochlorid**

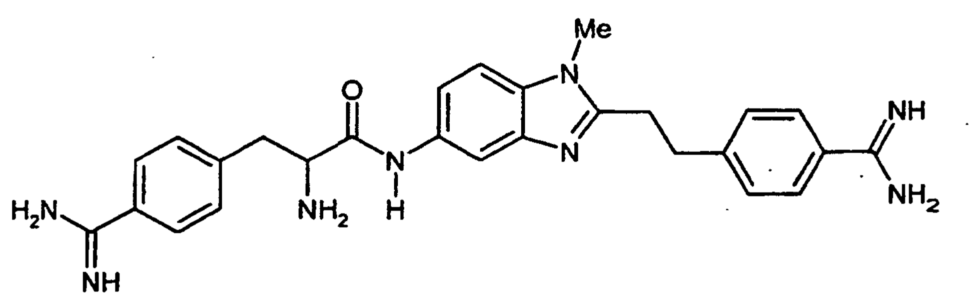

<u>a) N-{2-[2-(4-Cyanophenyl)-ethyl]-1-methyl-benzimidazol-5-yl}-[3-(*p*-cyanophenyl)-2-*t*-butyloxycarbonylamino-propionsäureamid]</u>

Die Synthese gelingt ausgehend von 10 mmol 5-Amino-2-[2-(4-cyanophenyl)-ethyl]-1-methyl-benzimidazol (Beispiel 1, Stufe d) und Boc-p-cyano-phenylalanin in Analogie zu der für Beispiel 1, Stufe f beschriebenen Vorgehensweise.
Ausbeute: 93%;

b) N-{2-[2-(4-Amidinophenyl)-ethyl]-1-methyl-benzimidazol-5-yl}-[2-amino-3-(*p*-amidinophenyl)-propionsäureamid]-trihydrochlorid

Die Synthese der Titelverbindung erfolgt wie für Beispiel 1, Stufe g beschrieben, ausgehend von N-{2-[2-(4-Cyanophenyl)-ethyl]-1-methyl-benzimidazol-5-yl}-[3-(*p*-cyanophenyl)-2-*t*-butyloxycarbonylamino-propionsäureamid] .
Schmp: > 260°C;
Masse: ber.: [482], gef.: $[M+H]^+$ 483, $[M+2H]^{2+}$ 242.
$^1$H-NMR (250MHz,DMSO-d6); 11.93 (1H,s,NH-C); 9.63; 9.32 (8H,2s, $-C(=NH_2+)NH_2$) 8.72 (3H,s,$NH_3^+$); 7.82-7.55 (11H,m,aryl-H); 4.58 (1H,m,-<u>CH</u>-$CH_2$); 3,94 (3H,s,N-$CH_3$); 6H,m,-CH-<u>$CH_2$</u>-;$CH_2$-$CH_2$-).

**<u>Beispiel 5:</u> N-{2-[2-(4-Amidinophenyl)-ethyl]-1-methyl-benzimidazol-5-yl}-N-isobutyl-[3-(4-amidinophenyl)-propionamid]-dihydrochlorid**

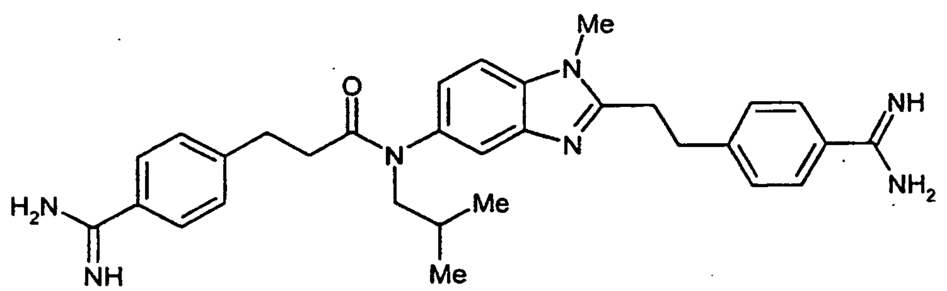

a) 2-[2-(4-Cyanophenyl)-ethyl]-1-methyl-5-isobutylamino-benzimidazol

1.0g (3.6 mmol) 5-Amino-2-[2-(4-cyanophenyl)-ethyl]-1-methyl-benzimidazol (erhältlich gemäß Beispiel 1, Stufe c) und Isobutyraldehyd (0.33 mL, 3.6 mmol) werden in 25 mL Dichlormethan mit 0,22 mL Essigsäure bei Raumtemperatur und unter Rühren mit $Na[HB(OAc)_3]$ versetzt. Es wird 1 h bei Raumtemperatur gerührt, mit Wasser überschichtet, vorsichtig mit konzentrierter, wässriger Salzsäure angesäuert und im Anschluß mit 4N NaOH-Lösung alkalisch gestellt. Die organische Phase wird abgetrennt, getrocknet und eingeengt. Das Produkt wird über Kieselgel chromatographiert und gegebenenfalls aus Diethylether kristallisiert.
Ausbeute: 0.8 g (67%); Schmp.: 112-114 °C.

b) N-{2-[2-(4-Cyanophenyl)-ethyl]-1-methyl-benzimidazol-5-yl}-N-isobutyl-[3-(4-cyanophenyl)-propionamid]

3-(*p*-Cyanophenyl)-propionsäure (0.69 g, 3.9 mmol) in 15 mL Chloroform wird mit 1.3 mL Thionylchlorid versetzt und 3 h unter Rückfluß erhitzt. Das Lösungsmittel sowie das überschüssige Thionylchlorid werden abdestilliert, der Rückstand in 10 mL Dichlormethan gelöst und zu einer Lösung von 2-[2-(4-Cyanophenyl)-ethyl]-1-methyl-5-isobutylamino-benzimidazol (1.25 g, 3.8 mmol) und 1.3 mL N-Methylmorpholin in 50 mL Dichlormethan zugetropft. Es wird 0.5h unter Rückfluß erhitzt, abgekühlt, mit Wasser versetzt und in die organische Phase extrahiert. Es wird mit verdünnter, wässriger NaOH und mit Wasser gewaschen, getrocknet und eingeengt. Das Produkt wird ohne Reinigung weiter umgesetzt.

c) N-{2-[2-(4-Amidinophenyl)-ethyl]-1-methyl-benzimidazol-5-yl}-N-isobutyl-[3-(4-amidinophenyl)-propionamid]-dihydrochlorid

N-{2-[2-(4-Cyanophenyl)-ethyl]-1-methyl-benzimidazol-5-yl}-N-isobutyl-[3-(4-cyanophenyl)-propionamid] (Rohprodukt erhalten gemäß Stufe b) wird in 25 mL einer gekühlten bei 0 °C gesättigten ethanolischen HCl-Lösung aufgenommen. Es wird bis zur vollständigen Auflösung des Eduktes gerührt und anschließend über Nacht bei 0-5 °C gehalten. Das Ethanol wird bei maximal 40 °C abdestilliert und der Rückstand in 30 mL einer bei 0 °C gesättigten ethanolischen Ammoniak-Lösung aufgenommen. Es wird 1 h bei Raumtemperatur, 2 h bei 40-50 °C gerührt, mit weiteren 10 mL gesAmmoniaklösung versetzt, 1 h unter Rückfluß gekocht und über Nacht bei Raumtemperatur gehalten. Der Alkohol wird abdestilliert, der Rückstand in Dichlormethan/Methanol = 4/1aufgeschlämmt, der unlösliche anorganische Rückstand abfiltriert und das Filtrat über Kieselgel chromatograpiert. Das Produkt wird aus Ethanol/Aceton mit wenig Wasser kristallisiert.

Ausbeute: 1.5 g (66%); Schmp.: > 220 °C;
Masse: ber.: [523], gef.: $[M+H]^+$ 524, $[M+Na]^+$ 546
$^1$H-NMR (250MHz, $CD_3OD$):δ=7.88-6.99 (11H, m, aryl-H); 3.78 (3H, s, N-$CH_3$); 3.58 (2H, d, J=7.4 Hz, $CH_2$-CH); 3.36 (4H, s, -$CH_2$-$CH_2$-); 2.98; 2.42 (4H, 2t, J=7.6 Hz, O=C-$CH_2CH_2$-); 1.72 (1H, m, CH-$(CH_3)_2$); 0.88 (6H, d, J=6.4 Hz, $(CH_3)_2$-CH).

**Beispiel 6: N-{2-[2-(4-Amidinophenyl)-ethyl]-1-methyl-benzimidazol-5-yl}-N-methyl-[3-(piperid-4-yl)-propionamid]-dihydrochlorid**

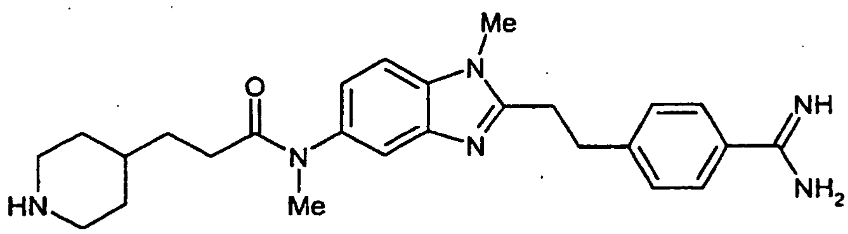

0.8g (1.3 mmol) N-{2-[2-(4-Amidinophenyl)-ethyl]-1-methyl-benzimidazol-5-yl}-N-methyl-[3-(N-benzyl-piperid-4-yl)-propionamid] (Beispiel 2) werden in 50 mL Mathanol in Gegenwart von 5 % Pd/C bei Normaldruck und 40-50 °C hydriert. Der Katalysator wird abfiltriert und das Filtrat eingeengt.
Ausbeute: 0.7 g (ca. 100%);
Masse: ber.: [446], gef.: $[M+H]^+$ 447.
$^1$H-NMR (250MHz, $CD_3OD$):δ=7.97-7.47 (7H, m, aryl-H); 3.98 (3H, s, N-$CH_3$) 3.62; 3.36 (4H, 2m, -$CH_2$-$CH_2$-); 3.34 (3H,s, O=C-N-$CH_3$); 2,88-1.09 (13H, m, Piperidinylethyl).

**Beispiel 7: N-{2-[2-(4-Amidinophenyl)-ethyl)-1-methyl-benzimidazol-5-yl}-N-methyl-[3-(piperid-4-yl)-propionamid]-dihydrochlorid**

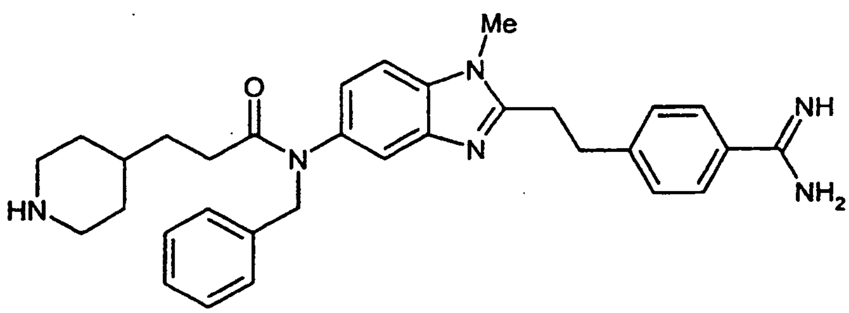

2,4 mmol N-{2-[2-(4-Amidinophenyl)-ethyl]-1-benzyl-benzimidazol-5-yl}-N-methyl-[3-(N-benzyl-piperid-4-yl)-propionamid] (Beispiel 3) werden analog zu der für Beispiel 6 beschriebenen Vorgehensweise zur Titelverbindung umgesetzt.
Ausbeute: 80%;
Masse: ber.: [522], gef.: $[M+H]^+$ 523, $[M+2H]^{2+}$ 262.
$^1$H-NMR (250MHz, $CD_3OD$):δ=7.94-7.16 (12H, m, aryl-H); 5.02 (2H, s, N-$CH_2$); 3.97 (3H, s, N-$CH_3$); 3.78; 3.24 (4H, 2m, -$CH_2$-$CH_2$-); 2.90-1.13 (13H, m, Piperidinylethyl).

**Beispiel 8: N-{2-[2-(4-Amidinophenyl)-ethyl]-1-methylbenzimidazol-5-yl}-N-(α-naphthylmethyl)-[3-amidinobenzylaminoacetamid]-trihydrochlorid**

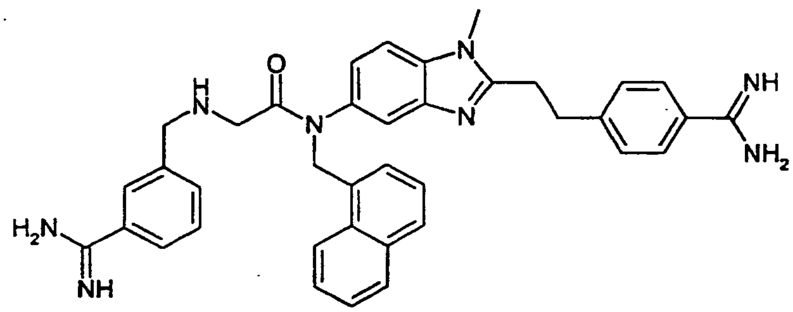

a) 2-[2-(4-Cyanophenyl)-ethyl]-1-methyl-5-(α-naphthylmethyl)-benzimidazol

5-Amino-2-[2-(4-cyanophenyl)-ethyl]-1-methyl-benzimidazol (35.5 g, 129.0 mmol) (erhältlich gemäß Beispiel 1, Stufe c) und 18.2 ml Naphthalin-1-carbaldehyd werden durch reduktive Aminierung gemäß Beispiel 5, Stufe a umgesetzt.
Ausbeute: 48.0 g (89%);

b) N-{2-[2-(4-Cyanophenyl)-ethyl]-1-methyl-benzimidazol-5-yl}-N-(α-naphthylmethyl)-[2-(N-tertbutyloxycarbonyl)-amin]-essigsäureamid

Die Synthese gelingt ausgehend von 3.7 g (8.9 mmol) 2-[2-(4-Cyanophenyl)-ethyl]-1-methyl-5-(α-naphthylmethylamino)-benzimidazol und 2.3 g (13.3 mmol) boc-Glycin in Analogie zu der für Beispiel 1, Stufe f beschriebenen Vorgehensweise.
Ausbeute: 4.0 g (80%).

c) N-{2-[2-(4-Cyanophenyl)-ethyl]-1-methylbenzimidazol-5-yl}-N-(α-naphthylmethyl)-2-(3-cyanobenzylamin)-essigsäureamid

2.7 g (5.7 mmol) N-{2-[2-(4-Cyanophenyl)-ethyl]-1-methyl-benzimidazol-5-yl}-N-(α-naphthylmethyl)-2-aminoessigsäureamid werden durch reduktive Aminierung laut Beispiel 1, Stufe c mit 0.82 g (6.3 mmol) 3-Cyanobenzaldehyd zur Titelverbindung umgesetzt.
Ausbeute: 2.6 g (88%).

d) N-{2-[2-(4-Amidinophenyl)-ethyl]-1-methylbenzimidazol-5-yl}-N-(α-naphthylmethyl)-[3-amidinobenzylaminoacetamid]-trihydrochlorid

Nach Beispiel 1, Stufe g wurden 1.3 g (2.2 mmol) N-{2-[2-(4-Cyanophenyl)-ethyl]-1-methylbenzimidazol-5-yl}-N-(α-naphthylmethyl)-2-(3-cyanobenzylamin)-essigsäureamid umgesetzt.
Ausbeute: 1.0 g (54%).
Masse: ber.: [622], gef.: $[M+H]^+$ 623.
$^1$H-NMR (250MHz, DMSO-d6): δ= 9.80 (1H, s, NH); 9.52 (1H, s, Amidin); 9.44 (1 H, s, Amidin); 9.36 (1H, s, Amidin); 9.23 (1 H, s, Amidin); 8.29-7.11 (18H, m, aryl-H); 6.50 (2H, s, $CH_2$); 4.24 (2H, s, $CH_2$); 3.86 (3H, s, $CH_3$); 3.67 (2H, s, $CH_2$); 3.46-3.23 (4H, m, $CH_2$-$CH_2$).

Die nachfolgende Tabelle faßt in Analogie zu den vorstehend beschriebenen Beispielen weitere, erfindungsgemäß synthetisierte Verbindungen der allgemeinen Formel (I) zusammen.

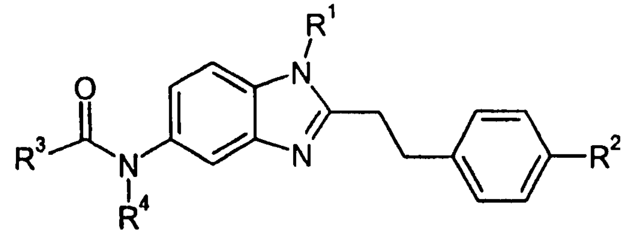

(I),

Tabelle 1:

| Nr | -R1 | -R2 | -R3 | -R4 | Chemische Bezeichnung |
|---|---|---|---|---|---|
| 9 | -Methyl | 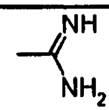 | 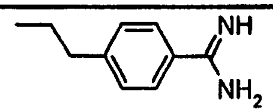 | 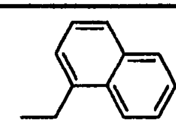 | N-{2-[2-(4-Amidinophenyl)-ethyl]-1-methyl-benzimidazol-5-yl}-N-α-naphthylmethyl-[3-(4-amidinophenyl)-propionamid]-dihydro-chlorid |
| 10 | -Methyl | 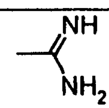 | 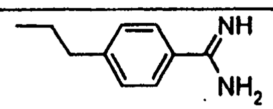 | 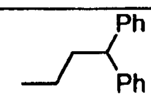 | N-{2-[2-(4-Amidinophenyl)-ethyl]-1-methyl-benzimidazol-5-yl}-N-(3,3-diphenylpropyl)-[3-(4-amidinophenyl)-propionamid]-dihydrochlorid |
| 11 | -Methyl | 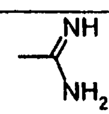 | 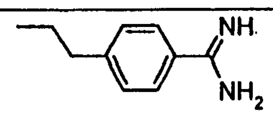 | 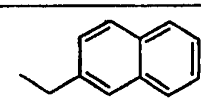 | N-{2-[2-(4-Amidinophenyl)-ethyl]-1-methyl-benzimidazol-5-yl}-N-β-naphthylmethyl-[3-(4-amidinophenyl)-propionamid]-dihydro-chlorid |

| Nr | -$R^1$ | -$R^2$ | -$R^3$ | -$R^4$ | Chemische Bezeichnung |
|---|---|---|---|---|---|
| 12 | -Methyl | 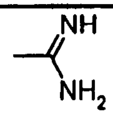 | 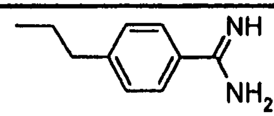 | -Benzyl | N-{2-[2-(4-Amidinophenyl)-ethyl]-1-methyl-benzimidazol-5-yl}-N-benzyl-[3-(4-amidino-phenyl)-propionamid]-dihydrochlorid |
| 13 | -Methyl | 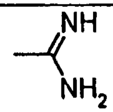 | 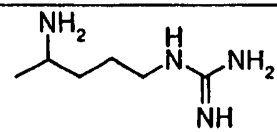 | Wasserstoff | N-{2-[2-(4-Amidinophenyl)-ethyl]-1-methyl-benzimidazol-5-yl}-[2-amino-5-guanidino-pentansäureamid]-trihydrochlorid |
| 14 | -Methyl | 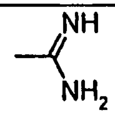 | 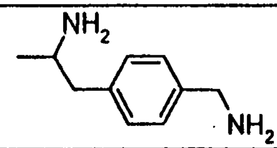 | Wasserstoff | N-{2-[2-(4-Amidinophenyl)-ethyl]-1-methyl-benzimidazol-5-yl}-[2-amino-3-[*p*-(amino-methyl)-phenyl]-propionsäureamid]-tri-hydrochlorid |
| 15 | -Methyl | 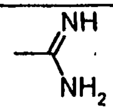 | 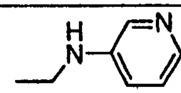 | -Methyl | N-{2-[2-(4-Amidinophenyl)-ethyl]-1-methyl-benzimidazol-5-yl}-N-methyl-[2-(pyrid-2-ylamino)-acetamid]-hydrochlorid |
| 16 | -Methyl | 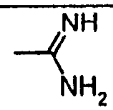 | 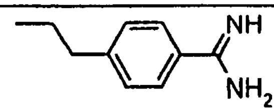 | 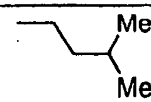 | N-{2-[2-(4-Amidinophenyl)-ethyl]-1-methyl-benzimidazol-5-yl}-N-isopentyl-[3-(4-amidi-nophenyl)-propionamid]-dihydrochlorid |
| 17 | -Methyl | 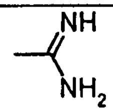 | 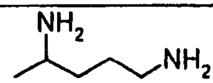 | Wasserstoff | N-{2-[2-(4-Amidinophenyl)-ethyl]-1-methyl-benzimidazol-5-yl}-[2,5-diamino-pentan-säureamid]-trihydrochlorid |
| 18 | -Methyl | 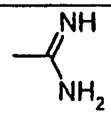 | 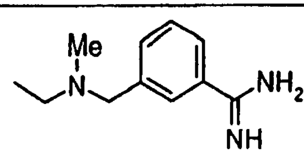 | 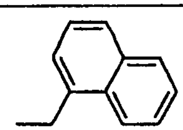 | N-{2-[2-(4-Amidinophenyl)-ethyl]-1-methyl-benzimidazol-5-yl}- N-(α-naphthylmethyl-amino)-N-methyl-N-[3-amidinobenzyl]-acetamid-trihydrochlorid |

| Nr | -R¹ | -R² | -R³ | -R⁴ | Chemische Bezeichnung |
|---|---|---|---|---|---|
| 19 | -Methyl | 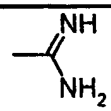 | 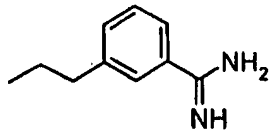 | 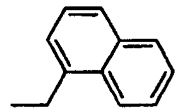 | N-{2-[2-(4-Amidinophenyl)-ethyl]-1-methyl-benzimidazol-5-yl}-N-α-naphthylmethyl-[3-(3-amidinophenyl)-propionamid]-dihydro-chlorid |
| 20 | -Methyl | 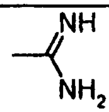 | 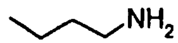 | 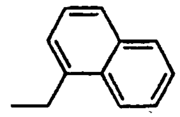 | N-{2-[2-(4-Amidinophenyl)-ethyl]-1-methyl-benzimidazol-5-yl}-N-α-naphthylmethyl-[4-amino-butansäureamid]-dihydrochlorid |
| 21 | -Methyl | 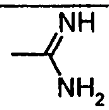 | 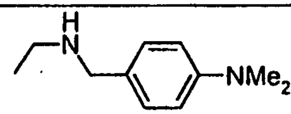 | 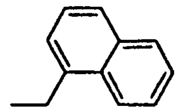 | N-{2-[2-(4-Amidinophenyl)-ethyl]-1-methyl-benzimidazol-5-yl}-N-(α-naphthyl)-[4-dimethylaminobenzylamino-acetamid]-dihydrochlorid |
| 22 | -Methyl | 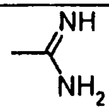 | 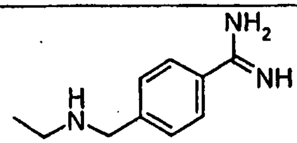 | 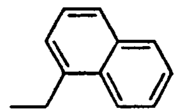 | N-{2-[2-(4-Amidinophenyl)-ethyl]-1-methyl-benzimidazol-5-yl}-N-(α-naphthylmethyl)-[4-amidinobenzylamino-acetamid]-trihydrochlorid |
| 23 | -Methyl | 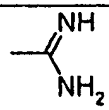 | 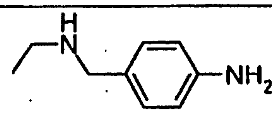 | 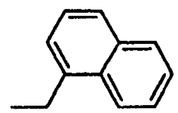 | N-{2-[2-(4-Amidinophenyl)-ethyl]-1-methyl-benzimidazol-5-yl}-N-(α-naphthylmethyl)-[4-aminobenzylamino-acetamid]-trihydrochlorid |
| 24 | -Methyl | 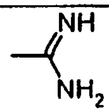 | 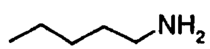 | 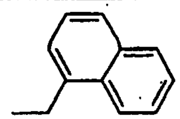 | N-{2-[2-(4-Amidinophenyl)-ethyl]-1-methyl-benzimidazol-5-yl}-N-(α-naphthylmethyl)-[5-amino-pentansäureamid]-dihydrochlorid |

| Nr | -R1 | -R2 | -R3 | -R4 | Chemische Bezeichnung |
|---|---|---|---|---|---|
| 25 | -Methyl | 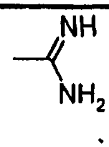 | 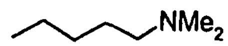 | 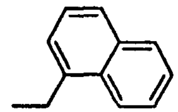 | N-{2-[2-(4-Amidinophenyl)-ethyl]-1-methyl-benzimidazol-5-yl}-N-α-naphthylmethyl-[5-dimethylamino-pentansäureamid]-dihydrochlorid |
| 26 | -Methyl | 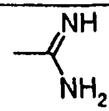 | 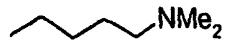 | 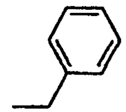 | N-{2-[2-(4-Amidinophenyl)-ethyl]-1-methyl-benzimidazol-5-yl}-N-benzyl-[5-dimethylamino-pentansäureamid]-dihydrochlorid |
| 27 | -Methyl | 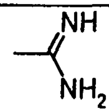 | 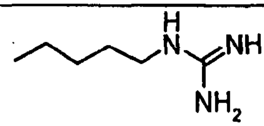 | 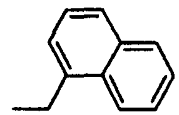 | N-{2-[2-(4-Amidinophenyl)-ethyl]-1-methyl-benzimidazol-5-yl}-N-(α-naphthylmethyl)-[5-guanidino-pentansäureamid]-dihydrochlorid |
| 28 | -Methyl | 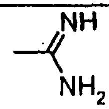 | 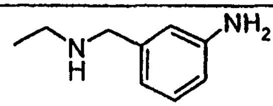 | 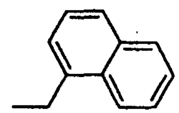 | N-{2-[2-(4-Amidinophenyl)-ethyl]-1-methyl-benzimidazol-5-yl}-N-(α-naphthylmethy)-[3-aminobenzylamino-acetamid]-trihydrochlorid |
| 29 | -Methyl | 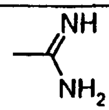 | 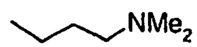 | 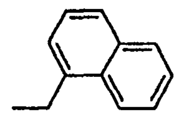 | N-{2-[2-(4-Amidinophenyl)-ethyl]-1-methyl-benzimidazol-5-yl}-N-(α-naphthylmethyl)-[4-dimethylamino-butansäureamid]-dihydrochlorid |
| 30 | -Methyl | 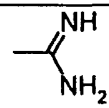 | 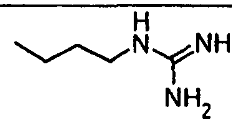 | 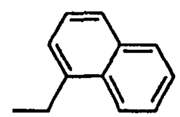 | N-{2-[2-(4-Amidinophenyl)-ethyl]-1-methyl-benzimidazol-5-yl}-N-(α-naphthylmethyl)-[4-guanidino-butansäureamid]-dihydrochlorid |

| Nr | -R1 | -R2 | -R3 | -R4 | Chemische Bezeichnung |
|---|---|---|---|---|---|
| 31 | -Methyl | 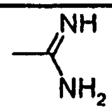 | 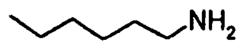 | 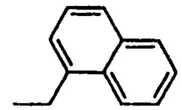 | N-{2-[2-(4-Amidinophenyl)-ethyl]-1-methyl-benzimidazol-5-yl}-N-(α-naphthylmethyl)-[6-amino-hexansäureamid]-dihydrochlorid |
| 32 | -Methyl | 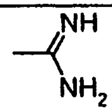 | 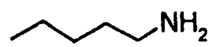 | 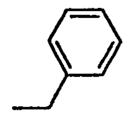 | N-{2-[2-(4-Amidinophenyl)-ethyl]-1-methyl-benzimidazol-5-yl}-N-benzyl-[5-amino-pentansäureamid]-dihydrochlorid |
| 33 | -Methyl | 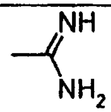 | 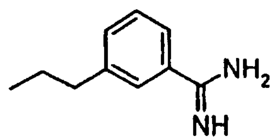 | 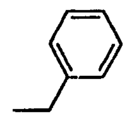 | N-{2-[2-(4-Amidinophenyl)-ethyl]-1-methyl-benzimidazol-5-yl}-N-benzyl-[3-(3-amidinophenyl)-propionamid]-dihydro-chlorid |
| 34 | -Methyl | 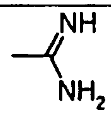 | 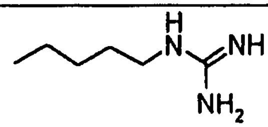 | 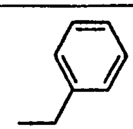 | N-{2-[2-(4-Amidinophenyl)-ethyl]-1-methyl-benzimidazol-5-yl}-N-benzyl-[5-guanidino-pentansäureamid]-dihydrochlorid |
| 35 | -Methyl | 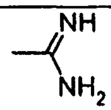 | 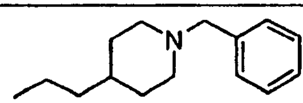 | 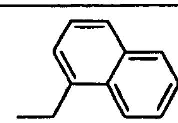 | N-{2-[2-(4-Amidinophenyl)-ethyl]-1-methyl-benzimidazol-5-yl}-N-(α-naphthylmethyl)-[3-(1-benzyl-piperidin-4-yl)-propionamid]-dihydrochlorid |
| 36 | -Methyl | 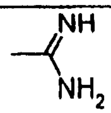 | 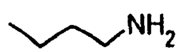 | 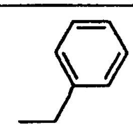 | N-{2-[2-(4-Amidinophenyl)-ethyl]-1-methyl-benzimidazol-5-yl}-N-benzyl-[4-amino-butansäureamid]-dihydrochlorid |

| Nr | -R1 | -R2 | -R3 | -R4 | Chemische Bezeichnung |
|---|---|---|---|---|---|
| 37 | -Methyl | 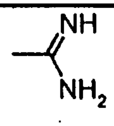 | 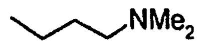 | 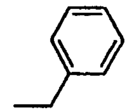 | N-{2-[2-(4-Amidinophenyl)-ethyl]-1-methyl-benzimidazol-5-yl}-N-benzyl-[4-dimethyl-amino-butansäureamid]-dihydrochlorid |
| 38 | -Methyl | 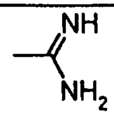 | 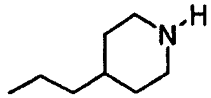 | 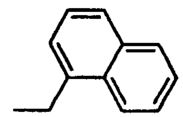 | N-{2-[2-(4-Amidinophenyl)-ethyl]-1-methyl-benzimidazol-5-yl}-N-(α-naphthylmethyl)-[3-(piperidin-4-yl)-propionamid]-dihydro-chlorid |
| 39 | -Methyl | 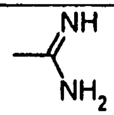 | 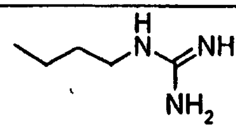 | 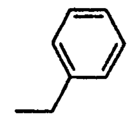 | N-{2-[2-(4-Amidinophenyl)-ethyl]-1-methyl-benzimidazol-5-yl}-N-benzyl-[4-guanidino-butansäureamid]-dihydrochlorid |
| 40 | -Methyl | 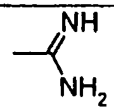 | 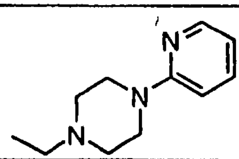 | 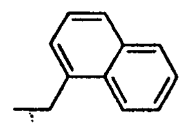 | N-{2-[2-(4-Amidinophenyl)-ethyl]-1-methyl-benzimidazol-5-yl}-N-(α-naphthylmethyl)-[2-(1-pyridin-2-yl-piperazin-4-yl)-acetamid]-dihydrochlorid |
| 41 | -Methyl | 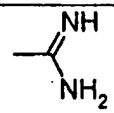 | 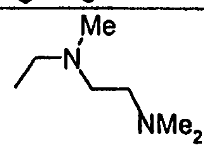 | 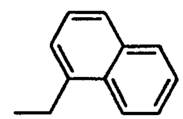 | N-{2-[2-(4-Amidinophenyl)-ethyl]-1-methyl-benzimidazol-5-yl}-N-(α-naphthylmethyl)-[2-(N-(2-dimethylaminoethyl)-N-methylamino)-acetamid]-trihydrochlorid |
| 42 | -Methyl | 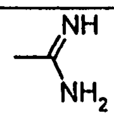 | 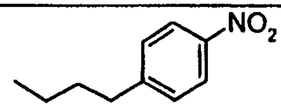 | 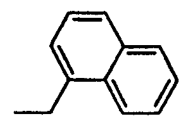 | N-{2-[2-(4-Amidinophenyl)-ethyl]-1-methyl-benzimidazol-5-yl}-N-(α-naphthylmethyl)-[4-(4-nitrophenyl)-butyroamid]-hydrochlorid |

| Nr | -$R^1$ | -$R^2$ | -$R^3$ | -$R^4$ | Chemische Bezeichnung |
|---|---|---|---|---|---|
| 43 | -Methyl | 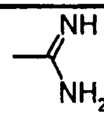 | 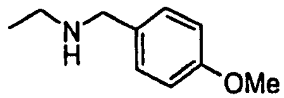 | 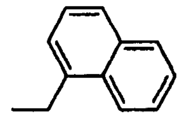 | N-{2-[2-(4-Amidinophenyl)-ethyl]-1-methyl-benzimidazol-5-yl}-N-(α-naphthylmethyl)-[2-(4-methoxybenzylamino)-acetamid]-dihydrochlorid |
| 44 | -Methyl | 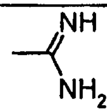 | 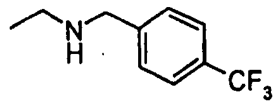 | 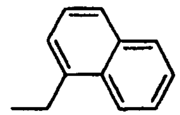 | N-{2-[2-(4-Amidinophenyl)-ethyl]-1-methyl-benzimidazol-5-yl}-N-(α-naphthylmethyl-amino)-[ 2-(4-trifluormethylbenzylamino)-acetamid]-diydrochlorid |
| 45 | -Methyl | 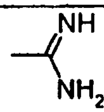 | 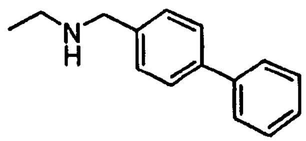 | 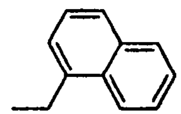 | N-{2-[2-(4-Amidinophenyl)-ethyl]-1-methyl-benzimidazol-5-yl}-N-(α-naphthylmethyl)-[2-(4-phenylbenzylamino)-acetamid]-dihydrochlorid |
| 46 | -Methyl | 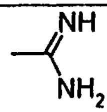 | 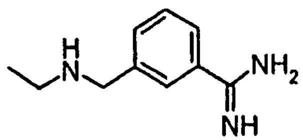 | 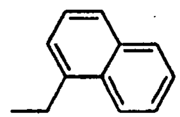 | N-{2-[2-(4-Amidinophenyl)-ethyl]-1-methyl-benzimidazol-5-yl}-N-(α-naphthylmethyl)-[2-(3-amidinobenzylamino)-acetamid]-trihydrochlorid |
| 47 | -Methyl | 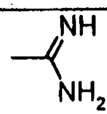 | 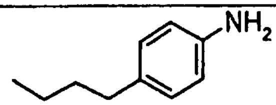 | 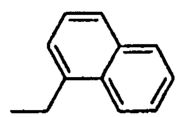 | N-{2-[2-(4-Amidinophenyl)-ethyl]-1-methyl-benzimidazol-5-yl}-N-(α-naphthylmethyl)-[4-(4-aminophenyl)-butyroamid]-dihydro-chlorid |
| 48 | -Methyl | 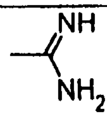 | 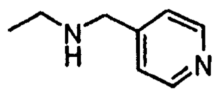 | 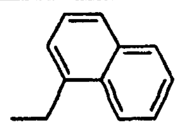 | N-{2-[2-(4-Amidinophenyl)-ethyl]-1-methyl-benzimidazol-5-yl}-N-(α-naphthylmethyl)-[2-(pyridin-4-ylmethylamino)-acetamid]-dihydrochlorid |

| Nr | -R1 | -R2 | -R3 | -R4 | Chemische Bezeichnung |
|---|---|---|---|---|---|
| 49 | -Methyl | 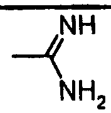 | 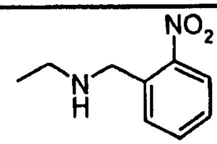 | 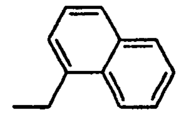 | N-{2-[2-(4-Amidinophenyl)-ethyl]-1-methyl-benzimidazol-5-yl}-N-(α-naphthylmethyl)-[2-(2-nitrobenzylamino)-acetamid]-dihydrochlorid |
| 50 | -Methyl | 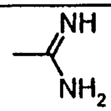 | 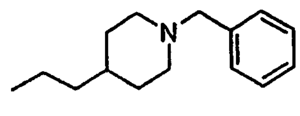 | 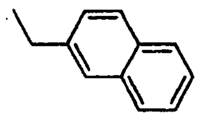 | N-{2-[2-(4-Amidinophenyl)-ethyl]-1-methyl-benzimidazol-5-yl}-N-(β-naphthylmethyl)-[3-(1-benzylpiperidin-4-yl)-propionamid]-dihydrochlorid |
| 51 | -Methyl | 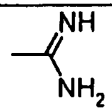 | 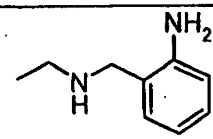 | 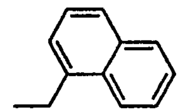 | N-{2-[2-(4-Amidinophenyl)-ethyl]-1-methyl-benzimidazol-5-yl}-N-(α-naphthylmethyl)-[2-(2-aminobenzylamino)-acetamid]-trihydrochlorid |
| 52 | -Methyl | 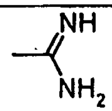 | 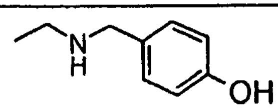 | 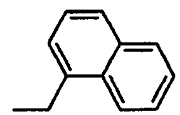 | N-{2-[2-(4-Amidinophenyl)-ethyl]-1-methyl-benzimidazol-5-yl}-N-(α-naphthylmethyl)-[2-(4-hydroxybenzylamino)-acetamid]-dihydrochlorid |
| 53 | -Methyl | 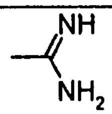 | 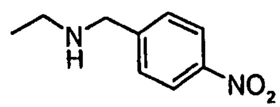 | 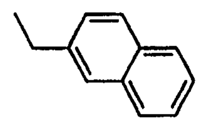 | N-{2-[2-(4-Amidinophenyl)-ethyl]-1-methyl-benzimidazol-5-yl}-N-(β-naphthylmethyl)-[2-(4-nitrobenzylamino)-acetamid]-hydrochlorid |
| 54 | -Methyl | 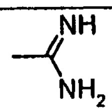 | 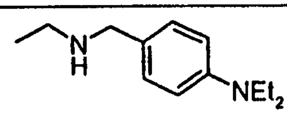 | 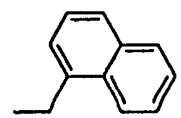 | N-{2-[2-(4-Amidinophenyl)-ethyl]-1-methyl-benzimidazol-5-yl}-N-(α-naphthylmethyl)-[2-(4-diethylaminobenzylamino)-acetamid]-dihydrochlorid |

| Nr | -R1 | -R2 | -R3 | -R4 | Chemische Bezeichnung |
|---|---|---|---|---|---|
| 55 | -Methyl | 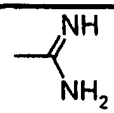 | 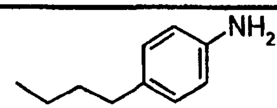 | 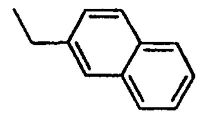 | N-{2-[2-(4-Amidinophenyl)-ethyl]-1-methyl-benzimidazol-5-yl}-N-(β-naphthylmethyl)-[4-(4-aminophenyl)-butyroamid]-dihydro-chlorid |
| 56 | -Methyl | 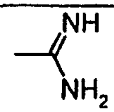 | 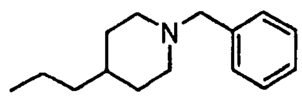 | 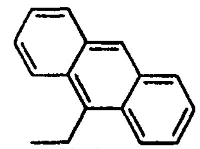 | N-{2-[2-(4-Amidinophenyl)-ethyl]-1-methyl-benzimidazol-5-yl}-N-(anthracen-9-ylmethyl)-[3-(1-benzyl-piperidin-4-yl)-propionamid]-dihydrochlorid |
| 57 | -Methyl | 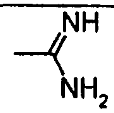 | 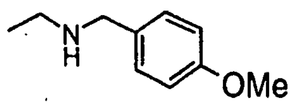 | 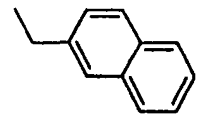 | N-{2-[2-(4-Amidinophenyl)-ethyl]-1-methyl-benzimidazol-5-yl}-N-(β-naphthylmethyl)-[4-methoxybenzylamino-acetamid]-dihydrochlorid |
| 58 | -Methyl | 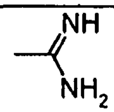 | 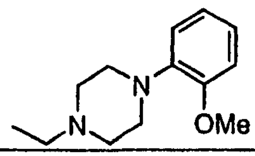 | 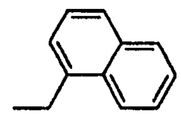 | N-{2-[2-(4-Amidinophenyl)-ethyl]-1-methyl-benzimidazol-5-yl}-N-(α-naphthylmethyl)-[2-(1-(2-methoxyphenyl)-piperazin-4-yl)-acetamid]-dihydrochlorid |
| 59 | -Methyl | 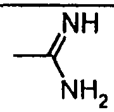 | 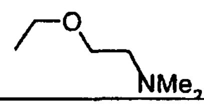 | 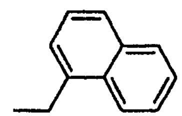 | N-{2-[2-(4-Amidinophenyl)-ethyl]-1-methyl-benzimidazol-5-yl}-N-(α-naphthylmethyl)-[2-(2-dimethylaminoethoxy)- acetamid]-dihydrochlorid |
| 60 | -Methyl | 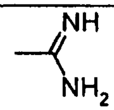 | 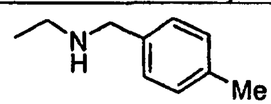 | 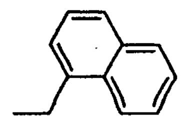 | N-{2-[2-(4-Amidinophenyl)-ethyl]-1-methyl-benzimidazol-5-yl}-N-(α-naphthylmethyl)-[2-(4-methylbenzylamino)-acetamid]-dihydrochlorid |

| Nr | -R1 | -R2 | -R3 | -R4 | Chemische Bezeichnung |
|---|---|---|---|---|---|
| 61 | -Methyl | 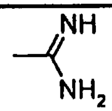 | 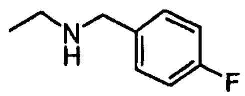 | 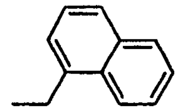 | N-{2-[2-(4-Amidinophenyl)-ethyl]-1-methyl-benzimidazol-5-yl}-N-(α-naphthylmethyl)-[2-(4-fluorbenzylamino)-acetamid]-dihydrochlorid |
| 62 | -Methyl | 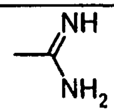 | 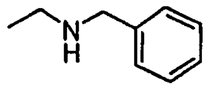 | 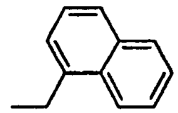 | N-{2-[2-(4-Amidinophenyl)-ethyl]-1-methyl-benzimidazol-5-yl}-N-(α-naphthylmethyl)-[2-(N-benzylamino)-acetamid]-dihydrochlorid |
| 63 | -Methyl | 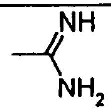 | 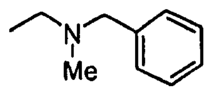 | 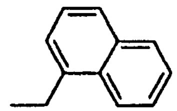 | N-{2-[2-(4-Amidinophenyl)-ethyl]-1-methyl-benzimidazol-5-yl}-N-(α-naphthylmethyl)-[2-(N-benzyl-N-methylamino)-acetamid]-dihydrochlorid |
| 64 | -Methyl | 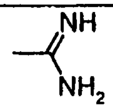 | 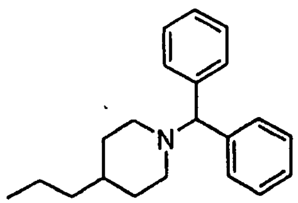 | 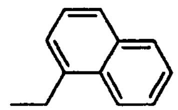 | N-{2-[2-(4-Amidinophenyl)-ethyl]-1-methyl-benzimidazol-5-yl}-N-(α-naphthylmethyl)-[3-(1-diphenylmethyl-piperidin-4-yl)-propionamid]-trihydrochlorid |
| 65 | -Methyl | 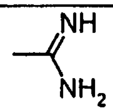 | 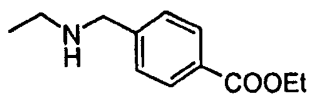 | 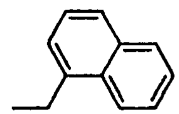 | N-{2-[2-(4-Amidinophenyl)-ethyl]-1-methyl-benzimidazol-5-yl}-N-(α-naphthylmethyl)-[2-(4-ethylcarboxylatbenzylamino)-acetamid]-dihydrochlorid |
| 66 | -Methyl | 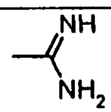 | 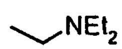 | 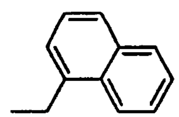 | N-{2-[2-(4-Amidinophenyl)-ethyl]-1-methyl-benzimidazol-5-yl}-N-(α-naphthylmethyl)-[2-diethylamino-acetamid]-dihydrochlorid |

| Nr | -R1 | -R2 | -R3 | -R4 | Chemische Bezeichnung |
|---|---|---|---|---|---|
| 67 | -Methyl | 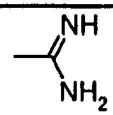 | 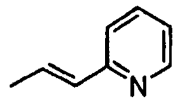 | 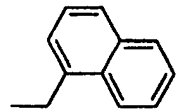 | N-{2-[2-(4-Amidinophenyl)-ethyl]-1-methyl-benzimidazol-5-yl}-N-(α-naphthylmethyl)-[3-(pyridin-2-yl)-acrylamid]-hydrochlorid |
| 68 | -Methyl | 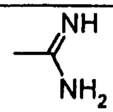 | 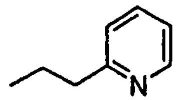 | 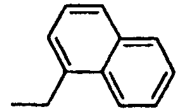 | N-{2-[2-(4-Amidinophenyl)-ethyl]-1-methyl-benzimidazol-5-yl}-N-(α-naphthylmethyl)-[3-(pyridin-2-yl)-propionamid]-hydrochlorid |
| 69 | -Methyl | 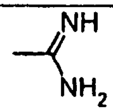 | 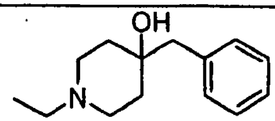 | 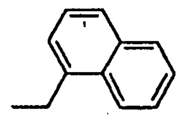 | N-{2-[2-(4-Amidinophenyl)-ethyl]-1-methyl-benzimidazol-5-yl}-N-(α-naphthylmethyl)-[2-(4-benzyl-4-hydroxypiperidin-1-yl)-acetamid]-dihydrochlorid |
| 70 | -Methyl | 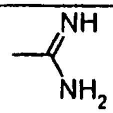 | 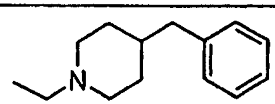 | 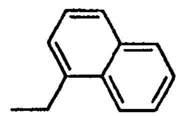 | N-{2-[2-(4-Amidinophenyl)-ethyl]-1-methyl-benzimidazol-5-yl}-N-(α-naphthylmethyl)-[2-(4-benzyl-4-piperidin-1-yl)-acetamid]-dihydrochlorid |
| 71 | -Methyl | 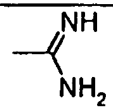 | 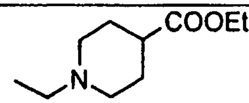 | 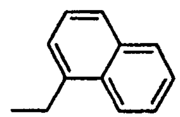 | N-{2-[2-(4-Amidinophenyl)-ethyl]-1-methyl-benzimidazol-5-yl}-N-(α-naphthylmethyl)-[2-(4-ethylcarboxylat-4-piperidin-1-yl)-acetamid]-dihydrochlorid |
| 72 | -Methyl | 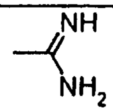 | 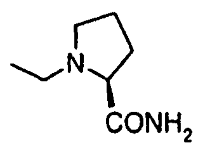 | 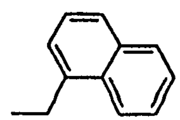 | N-{2-[2-(4-Amidinophenyl)-ethyl]-1-methyl-benzimidazol-5-yl}-N-(α-naphthylmethyl)-[2-(2-*S*-carbonsäureamid)-pyrrolidin-1-yl)-acetamid]-dihydrochlorid |

| Nr | -R1 | -R2 | -R3 | -R4 | Chemische Bezeichnung |
|---|---|---|---|---|---|
| 73 | -Methyl | 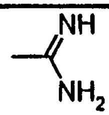 | 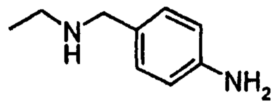 | 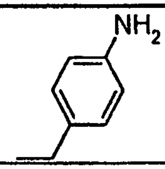 | N-{2-[2-(4-Amidinophenyl)-ethyl]-1-methyl-benzimidazol-5-yl}-N-(4-aminobenzyl)-[ 2-(4-aminobenzylamino)-acetamid]-dihydrochlorid |
| 74 | -Methyl | 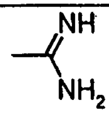 | 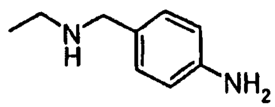 | 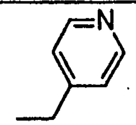 | N-{2-[2-(4-Amidinophenyl)-ethyl]-1-methyl-benzimidazol-5-yl}-N-(pyridin-4-ylmethyl)-[ 2-(4-aminobenzylamino)-acetamid]-dihydrochlorid |
| 75 | -Methyl | 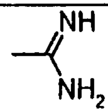 | 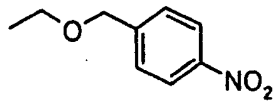 | 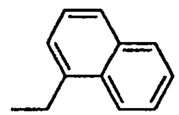 | N-{2-[2-(4-Amidinophenyl)-ethyl]-1-methyl-benzimidazol-5-yl}-N-(-α-naphthylmethyl)-[ 2-(4-nitrobenzyloxy)-acetamid]-hydrochlorid |
| 76 | -Methyl | 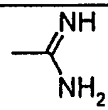 | 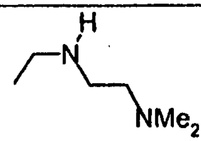 | 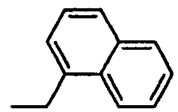 | N-{2-[2-(4-Amidinophenyl)-ethyl]-1-methyl-benzimidazol-5-yl}-N-(α-naphthylmethyl)-[ 2-(N-(2-dimethylaminoethyl)-amino)-acetamid]-trihydrochlorid |
| 77 | -Methyl | 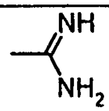 | 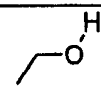 | 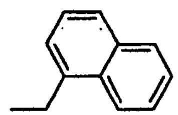 | N-{2-[2-(4-Amidinophenyl)-ethyl]-1-methyl-benzimidazol-5-yl}-N-(α-naphthylmethyl)-[3-hydroxy-acetamid]-hydrochlorid |
| 78 | -Methyl | 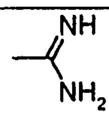 | 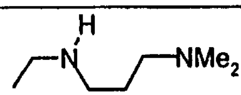 | 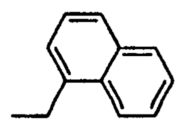 | N-{2-[2-(4-Amidinophenyl)-ethyl]-1-methyl-benzimidazol-5-yl}-N-(α-naphthylmethyl)-[ 2-(N-(3-dimethylaminopropyl)-amino)-acetamid]-trihydrochlorid |

| Nr | -R1 | -R2 | -R3 | -R4 | Chemische Bezeichnung |
|---|---|---|---|---|---|
| 79 | -Methyl | 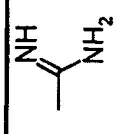 | 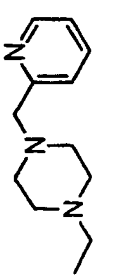 | 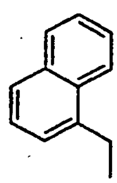 | N-{2-[2-(4-Amidinophenyl)-ethyl]-1-methyl-benzimidazol-5-yl}-N-(α-naphthylmethyl)-[2-(1-pyridin-2-yl-piperazin-4-yl)-acetamid]-trihydrochlorid |
| 80 | -Methyl | 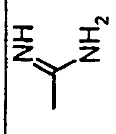 | 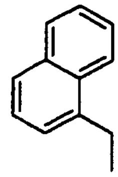 | 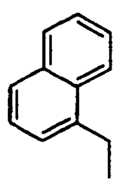 | N-{2-[2-(4-Amidinophenyl)-ethyl]-1-methyl-benzimidazol-5-yl}-N-(α-naphthylmethyl)-[2-(α-naphthyl)-acetamid]-hydrochlorid |
| 81 | -Methyl | 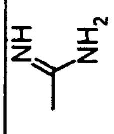 | 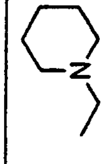 | 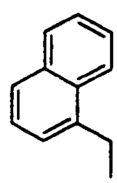 | N-{2-[2-(4-Amidinophenyl)-ethyl]-1-methyl-benzimidazol-5-yl}-N-(α-naphthylmethyl)-[2-(piperidin-1-yl)-acetamid]-dihydrochlorid |
| 82 | -Methyl | 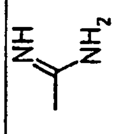 | 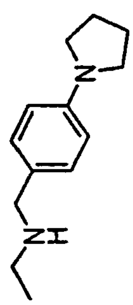 | 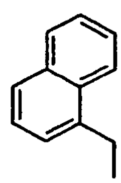 | N-{2-[2-(4-Amidinophenyl)-ethyl]-1-methyl-benzimidazol-5-yl}-N-(α-naphthylmethyl)-[2-(N-4-pyrrolidin-1-ylbenzylamino)-acetamid]-dihydrochlorid |
| 83 | -Methyl | 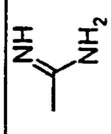 | 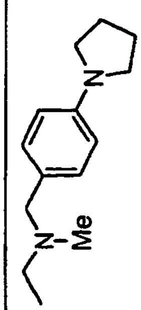 | 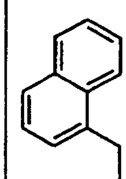 | N-{2-[2-(4-Amidinophenyl)-ethyl]-1-methyl-benzimidazol-5-yl}-N-(α-naphthylmethyl)-[2-(N-(4-pyrrolidin-1-ylbenzyl)-N-methylamino)-acetamid]-dihydrochlorid |

Die erfindungsgemäßen Verbindungen zeichnen sich durch ihre Tryptase-inhibierende Wirksamkeit aus. Besagte Fähigkeit, die Tryptase zu inhibieren, wurde gemäß der nachfolgenden Testbeschreibung untersucht.
Die Bestimmung wird in Tris HCl Puffer (100 mM), der zusätzlich Calcium (5 mM) und Heparin (100 mg/ml) enthält, bei pH 7.4 durchgeführt. Als Standard wird rh beta Tryptase eingesetzt, die beispielsweise von Prornega käuflich zu erwerben ist. Als Substrat dient N-p-Tosyl-Gly-Pro-Lys-para-nitroanilin in einer Konzentration von 0.6 mM. Das Substrat wird durch Tryptase verdaut wobei p-Nitroanilin entsteht, das bei 405 nm gemessen werden kann. Üblicherweise wird eine Inkubationszeit von 5 Minuten und eine Inkubationstemperatur von 37°C gewählt. Als Enzymaktivität werden 0.91 U/ml eingesetzt. Die Bestimmung erfolgt in einem Autoanalyser (Cobas Bio) der Firma Hofmann LaRoche. Die potentiellen Hemmsubstanzen werden in Konzentrationen von 10 µM im Screening eingesetzt, wobei die Hemmung der Tryptase in Prozent angegeben wird. Bei über 70 % Hemmung wird die $IC_{50}$ bestimmt (Konzentration bei der 50% der Enzymaktivität gehemmt ist). Nach 5-minütiger Vorinkubation der potentiellen Hemmsubstanzen, wird das Substrat zum Starten der Reaktion zugegeben, wobei die Bildung von p-Nitroanilin nach 5 Minuten, nach Testung der Linearität, als Maß für die Enzymaktivität genommen wird.

Die nach Durchführung des vorstehend beschriebenen Tests erhaltenen Daten (IC50-Werte) sind Tabelle 2 zu entnehmen.

Tabelle 2:

| Beispiel | $IC_{50}$ [µM] |
|---|---|
| 1 | 0,064 |
| 2 | 0,19 |
| 3 | 0,049 |
| 4 | 0,055 |
| 5 | 0,061 |
| 7 | 0,108 |
| 8 | 0,016 |
| 9 | 0,024 |
| 10 | 0,016 |
| 11 | 0,015 |
| 12 | 0,03 |
| 18 | 0,012 |
| 19 | 0,016 |
| 20 | 0,039 |
| 21 | 0,011 |
| 22 | 0,01 |
| 23 | 0,007 |
| 24 | 0,029 |
| 25 | 0,033 |
| 26 | 0,053 |
| 27 | 0,026 |
| 28 | 0,01 |
| 29 | 0,038 |
| 30 | 0,032 |
| 31 | 0,03 |
| 32 | 0,057 |
| 33 | 0,026 |
| 34 | 0,043 |

Tabelle 2: (fortgesetzt)

| Beispiel | $IC_{50}$ [µM] |
|---|---|
| 35 | 0,014 |
| 36 | 0,07 |
| 37 | 0,076 |
| 38 | 0,047 |
| 39 | 0,077 |
| 40 | 0,013 |
| 41 | 0,018 |
| 42 | 0,011 |
| 43 | 0,01 |
| 44 | 0,031 |
| 45 | 0,012 |
| 46 | 0,027 |
| 47 | 0,012 |
| 48 | 0,038 |
| 49 | 0,013 |
| 50 | 0,072 |
| 51 | 0,025 |
| 52 | 0,012 |
| 53 | 0,092 |
| 54 | 0,0086 |
| 55 | 0,172 |
| 56 | 0,0906 |
| 57 | 0,026 |
| 58 | 0,019 |
| 59 | 0,066 |
| 60 | 0,025 |
| 61 | 0,039 |
| 62 | 0,029 |
| 63 | 0,028 |
| 64 | 0,025 |
| 65 | 0,027 |
| 66 | 0,027 |
| 67 | 0,039 |
| 68 | 0,038 |
| 69 | 0,018 |
| 70 | 0,0039 |
| 71 | 0,0159 |

Tabelle 2: (fortgesetzt)

| Beispiel | $IC_{50}$ [µM] |
|---|---|
| 72 | 0,0584 |
| 73 | 0,0131 |
| 74 | 0,0574 |
| 75 | 0,0172 |
| 76 | 0,022 |
| 77 | 0,1096 |
| 78 | 0,0213 |
| 79 | 0,0201 |
| 80 | 0,0301 |
| 81 | 0,0631 |
| 82 | 0,0049 |
| 83 | 0,0076 |

Die erfindungsgemäßen Tryptase-Inhibitoren können oral, transdermal, inhalativ oder parenteral verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt

und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

Pharmazeutische Formulierungsbeispiele

A)

| Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B)

| Tabletten | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

C)

| Dragées | pro Dragee |
|---|---|
| Wirkstoff | 5 mg |
| Maisstärke | 41,5 mg |
| Milchzucker | 30 mg |
| Polyvinylpyrrolidon | 3 mg |
| Magnesiumstearat | 0.5 mg |
| | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragees werden mit Wachs poliert.

D)

| Kapseln | pro Kapsel |
|---|---|
| Wirkstoff | 50 mg |
| Maisstärke | 268,5 mg |
| Magnesiumstearat | 1,5 mg |
| | 320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

E)

| Ampullenlösung | |
|---|---|
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt, die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

F)

| Suppositorien | |
|---|---|
| Wirkstoff | 50 mg |
| Adeps solidus | 1650 mg |
| | 1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

## Patentansprüche

**1.** Verbindungen der allgemeinen Formel (I)

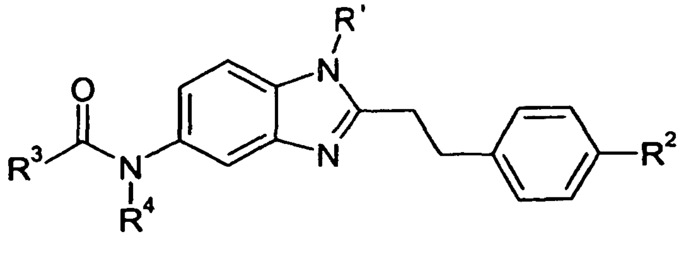

(I),

worin

$R^1$ $C_1$-$C_6$-Alkyl,

$R^2$ -C(=NH)$NH_2$

$R^3$ ein $C_1$-$C_6$-Alkyl-, $C_1$-$C_6$-Hydroxyalkyl- oder $C_1$-$C_4$-Alkoxy-$C_1$-$C_4$-alkyl-Rest, der ein- oder zweifach durch einen, zwei oder drei der Reste -$NR^5R^6$, -C(=NH)$NH_2$ oder -NH-C(=NH)$NH_2$ substituiert sein kann, oder
ein direkt, über eine $C_1$-$C_4$-Alkylen-Brücke oder eine $C_2$-$C_4$-Alkenylen-Brücke verknüpfter 5-, 6- oder 7-gliedriger, gesättigter oder ungesättigter Heterocyclus, der ein, zwei oder drei Heteroatome ausgewählt aus der Gruppe Sauerstoff, Stickstoff oder Schwefel enthalten kann, und der gegebenenfalls ein oder zweifach durch Hydroxy, $C_1$-$C_4$-Alkyl, -COO-$C_1$-$C_4$-Alkyl, -$CONH_2$, Benzyl, Diphenylmethyl, Phenyl oder Pyridylmethyl, Pyridyl substituiert sein kann, und wobei der Phenyl-Substituent ein-, zwei- oder dreifach durch einen oder mehrere der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$, substituiert sein kann, oder
Cyclopropyl, Cyclobutyl, Cyclopentyl oder Cyclohexyl, die jeweils ein- oder zweifach durch einen oder zwei der Reste -$NR^5R^6$, -C(=NH)$NH_2$ oder -NH-C(=NH)$NH_2$ substituiert sein können, oder
Phenyl-$C_1$-$C_4$-alkyl oder Naphthyl-$C_1$-$C_4$-alkyl, das an der Alkylenbrücke gegebenenfalls durch -$NR^5R^6$ substituiert sein kann und das am Phenylring jeweils ein- oder zweifach durch einen oder zwei der Reste -$NO_2$, -$NR^5R^6$, -$C_1$-$C_4$-Alkyl-$NR^5R^6$,
-C(=NH)$NH_2$ oder -NH-C(=NH)$NH_2$ substituiert sein kann,

$R^4$ Wasserstoff oder $C_1$-$C_6$-Alkyl, welches gegebenenfalls ein- oder zweifach durch einen oder zwei Reste ausgewählt aus der Gruppe Furanyl, Benzofuranyl, Thiophenyl, Benzothiophenyl, Anthracenyl, Phenyl, Pyridyl und Naphthyl substituiert sein kann, wobei die Substituenten Phenyl und Naphthyl ihrerseits jeweils ein-, zwei- oder dreifach durch einen oder mehrere der Reste ausgewählt aus der Gruppe $C_1$-$C_4$-Alkyl, $C_1$-$C_4$-Alkoxy, Halogen, -$C_1$-$C_4$-alkyl-Halogen, -$NH_2$,
-NH($C_1$-$C_4$-alkyl), -N($C_1$-$C_4$-alkyl)$_2$, $NO_2$, Hydroxy, -$CF_3$,
-NHCO-$C_1$-$C_4$-alkyl, -COOH, -COO($C_1$-$C_4$-alkyl), -$CONH_2$,
-CONH($C_1$-$C_4$-alkyl), -CON($C_1$-$C_4$-alkyl)$_2$,
-CONH($C_1$-$C_4$-alkyl)-COO($C_1$-$C_4$-alkyl) und Phenyl-$C_1$-$C_6$-alkyl substituiert sein können;

$R^5$ und $R^6$ gleich oder verschieden, Wasserstoff, $C_1$-$C_4$-Alkyl, Phenyl, Pyridyl oder Benzyl, welches gegebenenfalls durch einen Rest ausgewählt aus der Gruppe Halogen, Halo-$C_1$-$C_4$-Alkyl, -OH, -$C_1$-$C_4$-Alkyl, -O-$C_1$-$C_4$-alkyl, -CO-O-$C_1$-$C_4$-alkyl, -$NO_2$, Phenyl, Pyrrolidin-1-yl, Piperidin-1-yl, -$NH_2$, -NH-$C_1$-$C_4$-alkyl, -N($C_1$-$C_4$-alkyl)$_2$ und -C(=NH)$NH_2$ substituiert sein kann,

bedeuten können, mit der Maßgabe, dass Verbindungen der Formel 1, worin

$R^3$ eine $C_1$-$C_5$-Alkyl-, Phenyl-$C_1$-$C_3$-alkyl-, $C_3$-$C_6$-Cycloalkyl-, Pyridylgruppe, und
$R^4$ ein Wasserstoffatom, $C_1$-$C_5$ Alkyl oder Phenyl-$C_1$-$C_3$-alkylgruppe

bedeuten, ausgenommen sind, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**2.** Verbindungen der allgemeinen Formel (I) gemäß Anspruch 1, worin

$R^1$ $C_1$-$C_6$-Alkyl,

$R^2$ -C(=NH)$NH_2$;

$R^3$ ein $C_1$-$C_6$-Alkyl-Rest, der ein- oder zweifach durch einen oder zwei der Reste -$NR^5R^6$, -C(=NH)$NH_2$ oder -NH-C(=NH)$NH_2$ substituiert sein kann, oder
ein direkt oder über eine $C_1$-$C_4$-Alkylen-Brücke verknüpfter 5-, 6- oder 7-gliedriger, gesättigter oder ungesättigter Heterocyclus, der ein, zwei oder drei Heteroatome ausgewählt aus der Gruppe Sauerstoff, Stickstoff oder Schwefel enthalten kann und der gegebenenfalls ein oder zweifach durch

Hydroxy, $C_1$-$C_4$-Alkyl, Benzyl Phenyl oder Pyridyl substituiert sein kann, und wobei der Phenyl-Substituent durch einen der Reste ausgewählt aus der Gruppe $C_1$-$C_3$-Alkyl, $C_1$-$C_3$-Alkoxy, Halogen, Trifluormethyl und $-NH_2$, substituiert sein kann, oder
Cyclopropyl, Cyclopentyl oder Cyclohexyl, die jeweils ein- oder zweifach durch einen oder zwei der Reste $-NR^5R^6$, $-C(=NH)NH_2$ oder $-NH-C(=NH)NH_2$ substituiert sein können, oder
Phenyl-$C_1$-$C_4$-alkyl oder Naphthyl-$C_1$-$C_4$-alkyl, das an der Alkylenbrücke gegebenenfalls durch $-NR^5R^6$ substituiert sein kann und das am Phenylring jeweils ein- oder zweifach durch einen oder zwei der Reste $-NO_2$, $-NR^5R^6$, $-C_1$-$C_4$-Alkyl-$NR^5R^6$,
$-C(=NH)NH_2$ oder $-NH-C(=NH)NH_2$ substituiert sein kann,

$R^4$ Wasserstoff oder $C_1$-$C_6$-Alkyl, welches gegebenenfalls ein- oder zweifach durch einen oder zwei Reste ausgewählt aus der Gruppe Phenyl, Pyridyl und Naphthyl substituiert sein kann, wobei die Substituenten Phenyl und Naphthyl ihrerseits jeweils durch einen der Reste ausgewählt aus der Gruppe $C_1$-$C_4$-Alkyl, $C_1$-$C_4$-Alkoxy, Halogen, $-C_1$-$C_4$-alkyl-Halogen, $-NH_2$, substituiert sein können;

$R^5$ und $R^6$ gleich oder verschieden, Wasserstoff, $C_1$-$C_4$-Alkyl, Pyridyl oder Benzyl, welches gegebenenfalls durch einen Rest ausgewählt aus der Gruppe -OH, $-O-C_1$-$C_3$-alkyl, $-NO_2$, Phenyl, Pyrrolidin-1-yl, $-NH_2$, $-NH-C_1$-$C_4$-alkyl, $-N(C_1$-$C_4$-alykl$)_2$ und $-C(=NH)NH_2$ substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**3.** Verbindungen der allgemeinen Formel (I) gemäß Anspruch 1 oder 2, worin

$R^1$ $C_1$-$C_4$-Alkyl,

$R^2$ $-C(=NH)NH_2$;

$R^3$ ein $C_1$-$C_4$-Alkyl-Rest, der ein- oder zweifach durch einen oder zwei der Reste $-NR^5R^6$, $-C(=NH)NH_2$ oder $-NH-C(=NH)NH_2$ substituiert sein kann, oder
ein direkt oder über eine Methylen- oder Ethylen-Brücke verknüpfter 5-, 6- oder 7-gliedriger, gesättigter oder ungesättigter Heterocyclus, der ein oder zwei Heteroatome ausgewählt aus der Gruppe Sauerstoff oder Stickstoff enthalten kann und der gegebenenfalls durch Methyl oder Benzyl substituiert sein kann;
Naphthylmethyl, Benzyl oder Phenylethyl, die an der Alkylenbrücke gegebenenfalls durch $-NR^5R^6$ substituiert sein können und die am Phenylring durch einen Rest ausgewählt aus der Gruppe $-NR^5R^6$, $-C_1$-$C_4$-Alkyl-$NR^5R^6$, $-C(=NH)NH_2$ und $-NH-C(=NH)NH_2$ substituiert sein können,

$R^4$ Wasserstoff oder $C_1$-$C_5$-Alkyl, welches gegebenenfalls ein- oder zweifach durch einen oder zwei Reste ausgewählt aus der Gruppe Phenyl, Pyridyl und Naphthyl substituiert sein kann;

$R^5$ und $R^6$ gleich oder verschieden, Wasserstoff, Methyl, Ethyl, Propyl, Butyl, Pyridyl oder Benzyl, welches gegebenenfalls durch einen Rest ausgewählt aus der Gruppe-OH, Methoxy, $-NO_2$, Phenyl, Pyrrolidin-1-yl, $-NH_2$, -NH-Methyl, $-N(Methyl)_2$, -NH-Ethyl, $-N(Ethyl)_2$ und $-C(=NH)NH_2$ substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**4.** Verbindungen der allgemeinen Formel (I) gemäß Anspruch 1, 2 oder 3, worin

$R^1$ Methyl, Ethyl, Propyl oder Butyl;

$R^2$ $-C(=NH)NH_2$;

$R^3$ ein $C_2$-$C_4$-Alkyl-Rest, der ein- oder zweifach durch einen oder zwei der Reste $-NR^5R^6$, $-C(=NH)NH_2$ oder $-NH-C(=NH)NH_2$ substituiert sein kann, oder
ein über eine Methylen- oder Ethylen-Brücke verknüpfter 6-gliedriger, gesättigter oder ungesättigter Heterocyclus, der ein oder zwei Stickstoffatome enthält und der gegebenenfalls durch Methyl oder

Benzyl substituiert sein kann;
Naphthylmethyl, Benzyl oder Phenylethyl, die an der Alkylenbrücke gegebenenfalls durch $-NR^5R^6$ substituiert sein können und die am Phenylring durch einen Rest ausgewählt aus der Gruppe $-NR^5R^6$, $-C_1$-$C_4$-Alkyl-$NR^5R^6$, $-C(=NH)NH_2$ und $-NH-C(=NH)NH_2$ substituiert sind,

$R^4$ Wasserstoff oder ein Alkylrest ausgewählt aus der Gruppe Methyl, Ethyl, Propyl, Butyl und Pentyl, der gegebenenfalls ein- oder zweifach durch einen oder zwei Reste ausgewählt aus der Gruppe Phenyl, Pyridyl und Naphthyl substituiert sein kann;

$R^5$ und $R^6$ gleich oder verschieden, Wasserstoff, Methyl, Ethyl, Propyl, Butyl, Pyridyl oder Benzyl, welches gegebenenfalls durch einen Rest ausgewählt aus der Gruppe -OH, Methoxy, $-NO_2$, Phenyl, Pyrrolidin-1-yl, $-NH_2$, -NH-Methyl, $-N(Methyl)_2$ und
$-C(=NH)NH_2$ substituiert sein kann,
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**5.** Verbindungen der allgemeinen Formel (I) gemäß einem der Ansprüche1, 2, 3 oder 4, worin

$R^1$ Methyl, Ethyl oder Propyl;

$R^2$ $-C(=NH)NH_2$;

$R^3$ Methyl, das durch einen Rest ausgewählt aus der Gruppe Pyridylamino, Benzylamino, N-Benzyl-N-methyl-amino, N-(Amidinobenzyl)amino, N-(Amidinobenzyl)-N-methyl-amino, N-(Dimethylaminobenzyl)amino, (Pyrrolidin-1-ylbenzyl)amino, und N-(Dimethylaminobenzyl)-N-methyl-amino substituiert ist, oder
ein Alkyl-Rest ausgewählt aus der Gruppe Ethyl und Propyl, der ein- oder zweifach durch einen oder zwei Reste ausgewählt aus der Gruppe $-NH_2$ und $-NH-C(=NH)NH_2$ substituiert sein kann, oder
ein über eine Ethylen-Brücke verknüpfter Heterocyclus ausgewählt aus der Gruppe Piperidin, Morpholin und Piperazin, der gegebenenfalls durch Methyl Benzyl oder Diphenylmethyl substituiert sein kann;
Phenylethyl, das an der Ethylenbrücke gegebenenfalls durch $-NH_2$ substituiert sein kann und das am Phenylring durch einen Rest ausgewählt aus der Gruppe Pyrrolidin-1-yl, $-NH_2$, $-N(Methyl)_2$, $-CH_2-NH_2$ und $-C(=NH)NH_2$ substituiert ist;

$R^4$ Wasserstoff, Methyl, Ethyl, Propyl, Butyl, Pentyl, Benzyl, Naphthalinylmethyl, Pyridylmethyl oder Diphenylpropyl
bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**6.** Verbindungen der allgemeinen Formel (I) gemäß einem der Ansprüche 1, 2, 3, 4 oder 5, worin

$R^1$ Methyl, Ethyl oder Propyl;

$R^2$ $-C(=NH)NH_2$;

$R^3$ Methyl, das durch einen Rest ausgewählt aus der Gruppe N-(Amidinobenzyl)amino, N-(Amidinobenzyl)-N-methyl-amino, N-(Dimethylaminobenzyl)amino, (Pyrrolidin-1-ylbenzyl)amino, und N-(Dimethylaminobenzyl)-N-methyl-amino substituiert ist, oder
ein über eine Ethylen-Brücke verknüpftes Piperidin oder Piperazin, das gegebenenfalls durch Benzyl substituiert sein kann;
Phenylethyl, das an der Ethylenbrücke gegebenenfalls durch $-NH_2$ substituiert sein kann und das am Phenylring durch $-C(=NH)NH_2$ substituiert ist;

$R^4$ Wasserstoff, Methyl, Butyl, Benzyl, Naphthalinylmethyl oder Diphenylpropyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**7.** Verbindungen der allgemeinen Fonnel (IA)

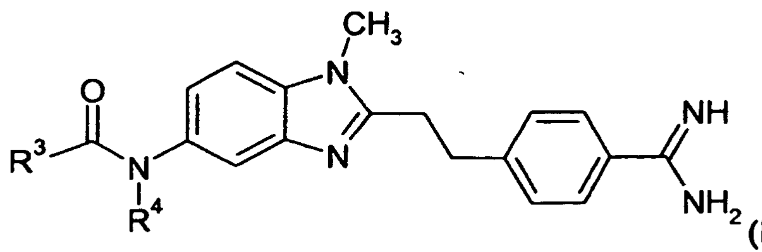

(IA),

worin

$R^3$ Methyl, das durch einen Rest ausgewählt aus der Gruppe N-(Amidinobenzyl)amino, N-(Amidinobenzyl)-N-methyl-amino, N-(Dimethylaminobenzyl)amino, (Pyrrolidin-1-ylbenzyl)amino, und N-(Dimethylaminobenzyl)-N-methyl-amino substituiert ist, oder
ein über eine Ethylen-Brücke verknüpftes Piperidin oder Piperazin, das gegebenenfalls durch Benzyl substituiert sein kann;
Phenylethyl, das an der Ethylenbrücke gegebenenfalls durch $-NH_2$ substituiert sein kann und das am Phenylring durch $-C(=NH)NH_2$ substituiert ist;

$R^4$ Wasserstoff, Methyl, Butyl, Benzyl, Naphthalinylmethyl oder Diphenylpropyl bedeuten können, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**8.** Prodrugs der allgemeinen Formel (II)

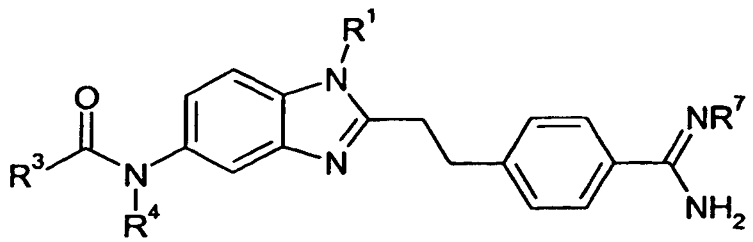

(II),

worin

$R^1$ und $R^4$ die vorstehend genannten Bedeutungen aufweisen können und

$R^3$ die vorstehend genannten Bedeutungen aufweisen kann oder $C_1$-$C_4$-Alkyl bedeutet, welches durch einen Rest ausgewählt aus der Gruppe
$-C(=NOH)NH_2$, $-C(=NCOO-C_1-C_{12}\text{-alkyl})NH_2$ oder
$-C(=NCOO-C_1-C_8\text{-alkyl-Phenyl})NH_2$ substituiert ist;

$R^7$ Hydroxy, $-COO-C_1-C_{12}$-Alkyl, -CO-Phenyl, -CO-Pyridyl oder
$-COO-C_1-C_8$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann,

bedeuten können, mit der Maßgabe, dass Verbindungen der Formel II, worin

$R^3$ eine $C_1$-$C_5$-Alkyl-, Phenyl-$C_1$-$C_3$-alkyl-, $C_3$-$C_6$-Cycloalkyl-, Pyridylgruppe, und
R4 ein Wasserstoffatom, $C_1$-$C_5$ Alkyl oder Phenyl-$C_1$-$C_3$-alkylgruppe

bedeuten, ausgenommen sind, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**9.** Prodrugs der allgemeinen Formel (II) gemäß Anspruch 8, worin

$R^1$ und $R^4$ die vorstehend genannten Bedeutungen aufweisen können und

$R^3$ die vorstehend genannten Bedeutungen aufweisen kann oder $C_1$-$C_4$-Alkyl bedeutet, welches durch einen Rest ausgewählt aus der Gruppe -C(=NOH)$NH_2$, -C(=NCOO-$C_1$-$C_6$-alkyl)$NH_2$ oder -C(=NCOO-$C_1$-$C_6$-alkyl-Phenyl)$NH_2$ substituiert ist;

$R^7$ Hydroxy, -COO-$C_1$-$C_6$-Alkyl, -CO-Phenyl, -CO-Pyridyl oder -COO-$C_1$-$C_6$-Alkyl-Phenyl, wobei in der vorstehend genannten Gruppe der Phenylring jeweils durch $C_1$-$C_4$-Alkyl, $C_1$-$C_4$-Alkoxy, OH, Halogen oder $CF_3$ substituiert sein kann, bedeuten kann, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**10.** Prodrugs der allgemeinen Formel (II) gemäß Anspruch 8 oder 9. worin

$R^1$, $R^3$ und $R^4$ die vorstehend genannten Bedeutungen aufweisen können und
$R^7$ Hydroxy, Methoxycarbonyl, Ethoxycarbonyl, Propyloxycarbonyl, Butyloxycarbonyl, Benzoyl, Benzyloxycarbonyl oder Nicotinoyl bedeuten kann,

gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

**11.** Verbindungen der allgemeinen Formel (III)

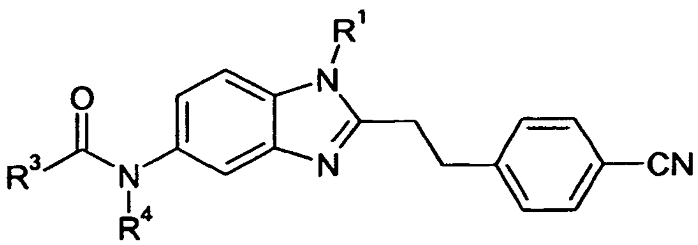

(III)

worin die Reste $R^1$, $R^3$ und $R^4$ die in den Ansprüchen 1-6 genannten Bedeutungen aufweisen können, mit der Maßgabe, dass Verbindungen der Formel III, worin

$R^3$ eine $C_1$-$C_5$-Alkyl-, Phenyl-$C_1$-$C_3$-alkyl-, $C_3$-$C_6$-Cycloalkyl-, Pyridylgruppe, und
R4 ein Wasserstoffatom, $C_1$-$C_5$ Alkyl oder Phenyl-$C_1$-$C_3$-alkylgruppe

bedeuten, ausgenommen sind,

**12.** Verwendung von Verbindungen der allgemeinen Formel (I) gemäß einem der Ansprüche 1-6 zur Herstellung eines Arzneimittels zur Vorbeugung und/oder Behandlung von Krankheiten, in denen Tryptase-Inhibitoren einen therapeutischen Nutzen entfalten können.

**13.** Verwendung von Prodrugs der allgemeinen Formel (II) gemäß einem der Ansprüche 8, 9 oder 10 zur Herstellung eines Arzneimittels zur Vorbeugung und/oder Behandlung von Krankheiten, in denen Tryptase-Inhibitoren einen therapeutischen Nutzen entfalten können.

**14.** Pharmazeutische Zusammensetzung **gekennzeichnet durch** einen Gehalt einer oder mehrer Verbindungen gemäß einem der Ansprüche 1-10.

**15.** Verfahren zur Herstellung von Verbindungen der allgemeinen Formel (I)

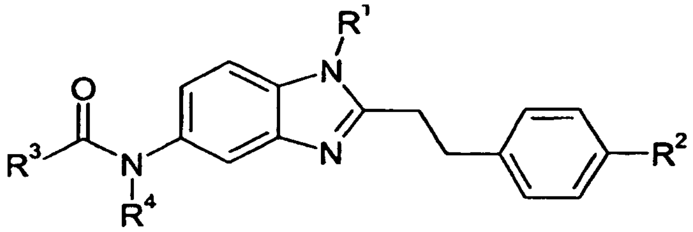

(I),

worin die Reste $R^1$, $R^2$, $R^3$ und $R^4$ die in den Ansprüchen 1-6 genannten Bedeutungen haben können, mit der Maßgabe, dass Verbindungen der Formel I, worin

$R^3$ eine $C_1$-$C_5$-Alkyl-, Phenyl-$C_1$-$C_3$-alkyl-, $C_3$-$C_6$-Cycloalkyl-, Pyridylgruppe, und
R4 ein Wasserstoffatom, $C_1$-$C_5$ Alkyl oder Phenyl-$C_1$-$C_3$-alkylgruppe

bedeuten, ausgenommen sind, **dadurch gekennzeichnet, dass** eine Verbindung der allgemeinen Formel (III)

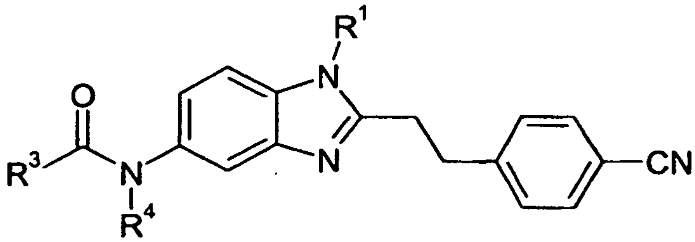

(III)

in der die Reste $R^1$, $R^3$ und $R^4$ die in den Ansprüchen 1-6 genannten Bedeutungen haben können, direkt in die Verbindungen der allgemeinen Formel (I) überführt wird.

**Claims**

**1.** Compounds of general formula (I)

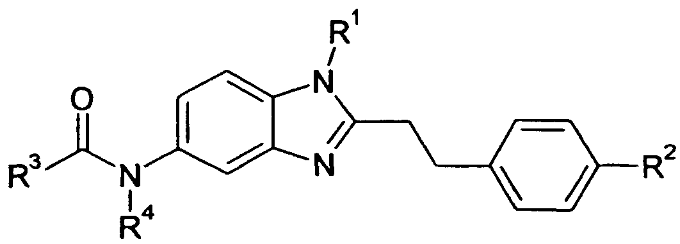

(I),

wherein

$R^1$ denotes $C_1$-$C_6$-alkyl

$R^2$ denotes -C(=NH)$NH_2$

$R^3$ denotes a $C_1$-$C_6$-alkyl, $C_1$-$C_6$-hydroxyalkyl or $C_1$-$C_4$-alkoxy-$C_1$-$C_4$-alkyl group which may be mono- or disubstituted by one, two or three of the groups -$NR^5R^6$, -C(=NH)$NH_2$ or-NH-C(=NH)$NH_2$, or a 5-, 6- or 7-membered, saturated or unsaturated heterocycle linked directly or via a $C_1$-$C_4$-alkylene bridge or via a $C_2$-$C_4$-alkenylene bridge, which may contain one, two or three heteroatoms selected from among oxygen, nitrogen or sulphur and which may optionally be mono- or disubstituted by hydroxy, $C_1$-$C_4$-alkyl, -COO-$C_1$-$C_4$-alkyl, -$CONH_2$, benzyl, diphenylmethyl, phenyl or pyridylmethyl, pyridyl, and wherein the phenyl substituent may be mono-, di- or trisubstituted by one or more groups

selected from among $C_1$-$C_4$-alkyl, $C_1$-$C_4$-alkoxy, halogen, $C_1$-$C_4$-alkyl-halogen and $-NH_2$, or cyclopropyl, cyclobutyl, cyclopentyl or cyclohexyl, each of which may be mono- or disubstituted by one or two of the groups $-NR^5R^6$, $-C(=NH)NH_2$ or $-NH-C(=NH)NH_2$, or phenyl-$C_1$-$C_4$-alkyl or naphthyl- $C_1$-$C_4$-alkyl, which may optionally be substituted at the alkylene bridge by $-NR^5R^6$ and may be mono- or disubstituted at the phenyl ring by one or two of the groups $-NO_2$, $-NR^5R^6$, $-C_1$-$C_4$-alkyl-$NR^5R^6$, $-C(=NH)NH_2$ or $-NH-C(=NH)NH_2$,

$R^4$ denotes hydrogen or $C_1$-$C_6$-alkyl, which may optionally be mono- or disubstituted by one or two groups selected from among furanyl, benzofuranyl, thiophenyl, benzothiophenyl, anthracenyl, phenyl, pyridyl and naphthyl, while the substituents phenyl and naphthyl may in turn be mono-, di- or trisubstituted by one or more of the groups selected from among $C_1$-$C_4$-alkyl, $C_1$-$C_4$-alkoxy, halogen, $C_1$-$C_4$-alkyl-halogen, $-NH_2$, $-NH(C_1$-$C_4$-alkyl), $-N(C_1$-$C_4$-alkyl$)_2$, $NO_2$, hydroxy, $-CF_3$, $-NHCO-C_1$-$C_4$-alkyl, -COOH, $-COO(C_1$-$C_4$-alkyl), $-CONH_2$, $-CONH(C_1$-$C_4$-alkyl), $-CON(C_1$-$C_4$-alkyl$)_2$, $-CONH(C_1$-$C_4$-alkyl)-$COO(C_1$-$C_4$-alkyl) and phenyl-$C_1$-$C_6$-alkyl;

$R^5$ and $R^6$, which may be identical or different, denote hydrogen, $C_1$-$C_4$-alkyl, phenyl, pyridyl or benzyl, which may optionally be substituted by a group selected from among halogen, halo-$C_1$-$C_4$-alkyl, -OH, $C_1$-$C_4$-alkyl , $-O-C_1$-$C_4$-alkyl, $-CO-O-C_1$-$C_4$-alkyl, $-NO_2$, phenyl, pyrrolidin-1-yl, piperidin-1-yl, $-NH_2$, $-NH-C_1$-$C_4$-alkyl, $-N(C_1$-$C_4$-alkyl$)_2$ and $-C(=NH)NH_2$ $-NH_2$,

with the proviso that compounds of formula I wherein

$R^3$ denotes a $C_1$-$C_5$-alkyl, phenyl- $C_1$-$C_3$-alkyl, $C_3$-$C_6$-cycloalkyl or pyridyl group and

$R^4$ denotes a hydrogen atom, a $C_1$-$C_5$-alkyl or phenyl- $C_1$-$C_3$-alkyl group

are excluded,
optionally in the form of the tautomers, the racemates, the enantiomers, the diastereomers and the mixtures thereof, and optionally the pharmacologically acceptable acid addition salts thereof.

**2.** Compounds of general formula (I) according to Claim 1 wherein

$R^1$ denotes $C_1$-$C_6$-alkyl,

$R^2$ denotes $-C(=NH)NH_2$;

$R^3$ denotes a $C_1$-$C_6$-alkyl group, which may be mono- or disubstituted by one or two of the groups $-NR^5R^6$, $-C(=NH)NH_2$ or $-NH-C(=NH)NH_2$, or a 5-, 6- or 7-membered, saturated or unsaturated heterocycle linked directly or via a $C_1$-$C_4$-alkylene bridge, which may contain one, two or three heteroatoms selected from among oxygen, nitrogen or sulphur and may optionally be mono- or disubstituted by hydroxy, $C_1$-$C_4$-alkyl, benzyl, phenyl or pyridyl, and wherein the phenyl substituent may be substituted by one of the groups selected from among $C_1$-$C_3$-alkyl, $C_1$-$C_3$-alkoxy, halogen, trifluoromethyl and $NH_2$, or cyclopropyl, cyclopentyl or cyclohexyl, each of which may be mono- or disubstituted by one or two of the groups $-NR^5R^6$, $-C(=NH)NH_2$ or $-NH-C(=NH)NH_2$, or phenyl-$C_1$-$C_4$-alkyl or naphthyl-$C_1$-$C_4$-alkyl, which may optionally be substituted by $-NR^5R^6$ at the alkylene bridge and may be mono- or disubstituted at the phenyl ring by one or two of the groups $-NO_2$, $-NR^5R^6$) $-C_1$-$C_4$-Alkyl-$NR^5R^6$, $-C(=NH)NH_2$ or-NH- $C(=NH)NH_2$,

$R^4$ denotes hydrogen or $C_1$-$C_6$-alkyl, which may optionally be mono- or disubstituted by one or two groups selected from among phenyl, pyridyl and naphthyl, wherein the substituents phenyl and naphthyl may in turn be substituted by one of the groups selected from among $C_1$-$C_4$-alkyl, $C_1$-$C_4$-alkoxy, halogen, $-C_1$-$C_4$-alkyl-halogen, $-NH_2$;

$R^5$ and $R^6$, which may be identical or different, denote hydrogen, $C_1$-$C_4$-alkyl, pyridyl or benzyl, which may optionally be substituted by a group selected from among-OH, $-O-C_1$-$C_3$-alkyl, $-NO_2$, phenyl, pyrrolidin-1-yl, $-NH_2$, $-NH-C_1$-$C_4$-alkyl, $-N(C_1$-$C_4$-alkyl$)_2$ and $-C(=NH)NH_2$,

optionally in the form of the tautomers, the racemates, the enantiomers, the diastereomers and the mixtures thereof, and optionally the pharmacologically acceptable acid addition salts thereof.

**3.** Compounds of general formula (I) according to Claim 1 or 2, wherein

$R^1$ denotes $C_1$-$C_4$-alkyl,

$R^2$ denotes $-C(=NH)NH_2$;

$R^3$ denotes a $C_1$-$C_4$-alkyl group, which may be mono- or disubstituted by one or two of the groups $-NR^5R^6$, $-C(=NH)NH_2$ or $-NH-C(=NH)NH_2$, or
a 5-, 6- or 7-membered, saturated or unsaturated heterocycle linked directly or via a methylene or ethylene bridge, which may contain one or two heteroatoms selected from among oxygen or nitrogen and may optionally be substituted by methyl or benzyl;
naphthylmethyl, benzyl or phenylethyl, which may optionally be substituted by $-NR^5R^6$ at the alkylene bridge and may be substituted at the phenyl ring by a group selected from among $-NR^5R^6$, $-C_1$-$C_4$-alkyl-$NR^5R^6$, $-C(=NH)NH_2$ and $-NH-C(=NH)NH_2$,

$R^4$ denotes hydrogen or $C_1$-$C_5$-alkyl, which may optionally be mono- or disubstituted by one or two groups selected from among phenyl, pyridyl and naphthyl;

$R^5$ and $R^6$, which may be identical or different, denote hydrogen, methyl, ethyl, propyl, butyl, pyridyl or benzyl, which may optionally be substituted by a group selected from among -OH, methoxy, $-NO_2$, phenyl, pyrrolidin-1-yl, $-NH_2$, -NH-methyl, -N(methyl)$_2$, -NH-ethyl, - N(ethyl)$_2$ and $-C(=NH)NH_2$,

optionally in the form of the tautomers, the racemates, the enantiomers, the diastereomers and the mixtures thereof, and optionally the pharmacologically acceptable acid addition salts thereof.

**4.** Compounds of general formula (I) according to Claim 1, 2 or 3, wherein

$R^1$ denotes methyl, ethyl, propyl or butyl;

$R^2$ denotes $-C(=NH)NH_2$;

$R^3$ denotes a $C_2$-$C_4$-alkyl group, which may be mono- or disubstituted by one or two of the groups $-NR^5R^6$, $-C(=NH)NH_2$ or $-NH-C(=NH)NH_2$, or
a 6-membered, saturated or unsaturated heterocycle linked via a methylene or ethylene bridge, which contains one or two nitrogen atoms and may optionally be substituted by methyl or benzyl;
naphthylmethyl, benzyl or phenylethyl, which may optionally be substituted by $-NR^5R^6$ at the alkylene bridge and which are substituted at the phenyl ring by a group selected from among $-NR^5R^6$, $-C_1$-$C_4$-alkyl-$NR^5R^6$, $-C(=NH)NH_2$ and $-NH-C(=NH)NH_2$,

$R^4$ denotes hydrogen or an alkyl group selected from among methyl, ethyl, propyl, butyl and pentyl, which may optionally be mono- or disubstituted by one or two groups selected from among phenyl, pyridyl and naphthyl;

$R^5$ and $R^6$, which may be identical or different, denote hydrogen, methyl, ethyl, propyl, butyl, pyridyl or benzyl, which may optionally be substituted by a group selected from among -OH, methoxy, $-NO_2$, phenyl, pyrrolidin-1-yl, $-NH_2$, -NH-methyl, -N(methyl)$_2$ and $-C(=NH)NH_2$,

optionally in the form of the tautomers, the racemates, the enantiomers, the diastereomers and the mixtures thereof, and optionally the pharmacologically acceptable acid addition salts thereof.

**5.** Compounds of general formula (I) according to one of claims 1, 2, 3 or 4, wherein

$R^1$ denotes methyl, ethyl or propyl;

$R^2$ denotes $-C(=NH)NH_2$;

$R^3$ denotes methyl which is substituted by a group selected from among pyridylamino, benzylamino, N-benzyl-N-methylamino, N-(amidinobenzyl)amino, N-(amidinobenzyl)-N-methyl-amino, N-(dimethylaminobenzyl)amino, (pyrrolidin-1-ylbenzyl)amino and N-(dimethylaminobenzyl)-N-methyl-amino, or
an alkyl group selected from among ethyl and propyl, which may be mono- or disubstituted by one or two groups selected from among $-NH_2$ and $-NH-C(=NH)NH_2$, or
a heterocycle linked via an ethylene bridge, selected from among piperidine, morpholine and piperazine, which may optionally be substituted by methyl, benzyl or diphenylmethyl;
phenylethyl, which may optionally be substituted by $-NH_2$ at the ethylene bridge and is substituted at the phenyl ring by a group selected from among pyrrolidin-1-yl, $-NH_2$, $-N(methyl)_2$, $-CH_2-NH_2$ and $-C(=NH)NH_2$;

$R^4$ denotes hydrogen, methyl, ethyl, propyl, butyl, pentyl, benzyl, naphthalinylmethyl or diphenylpropyl,

optionally in the form of the tautomers, the racemates, the enantiomers, the diastereomers and the mixtures thereof, and optionally the pharmacologically acceptable acid addition salts thereof.

**6.** Compounds of general formula (I) according to one of claims 1, 2, 3, 4 or 5, wherein

$R^1$ denotes methyl, ethyl or propyl;

$R^2$ denotes $-C(=NH)NH_2$;

$R^3$ denotes methyl, which is substituted by a group selected from among N-(amidinobenzyl)amino, N-(amidinobenzyl)-N-methyl-amino, N-(dimethylaminobenzyl) amino, (pyrrolidin-1-ylbenzyl) amino and N-(dimethylaminobenzyl)-N-methyl-amino, or
a piperidine or piperazine linked via an ethylene bridge, which may optionally be substituted by benzyl;
phenylethyl, which may optionally be substituted by $-NH_2$ at the ethylene bridge and is substituted by $-C(=NH)NH_2$ at the phenyl ring;

$R^4$ may denote hydrogen, methyl, butyl, benzyl, naphthalinylmethyl or diphenylpropyl,

optionally in the form of the tautomers, the racemates, the enantiomers, the diastereomers and the mixtures thereof, and optionally the pharmacologically acceptable acid addition salts thereof.

**7.** Compounds of general formula (IA)

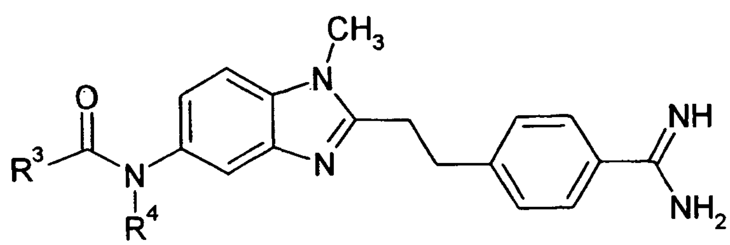
(IA),

wherein

$R^3$ denotes methyl which is substituted by a group selected from among N-(amidinobenzyl)amino, N-(amidinobenzyl)-N-methyl-amino, N-(dimethylaminobenzyl)amino, (pyrrolidin-1-ylbenzyl)amino, and N-(dimethylaminobenzyl)-N-methyl-amino, or
a piperidine or piperazine linked via an ethylene bridge, which may optionally be substituted by benzyl;
phenylethyl which may optionally be substituted by $-NH_2$ at the ethylene bridge and which is substituted at the phenyl ring by $-C(=NH)NH_2$;

$R^4$ denotes hydrogen, methyl, butyl, benzyl, naphthalinylmethyl or diphenylpropyl,

optionally in the form of the tautomers, the racemates, the enantiomers, the diastereomers and the mixtures thereof, and optionally the pharmacologically acceptable acid addition salts thereof.

**8.** Prodrugs of general formula (II)

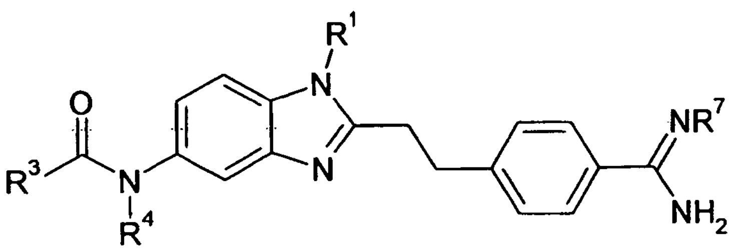

(II),

wherein

$R^1$ and $R^4$ may have the meanings given hereinbefore and

$R^3$ may be as hereinbefore defined or may denote $C_1$-$C_4$-alkyl , which is substituted by a group selected from among -C(=NOH)$NH_2$,
-C(=NCOO-$C_1$-$C_{12}$-alkyl)$NH_2$ or
-C(=NCOO-$C_1$-$C_8$-alkyl-phenyl)$NH_2$;

$R^7$ may denote hydroxy, -COO-$C_1$-$C_{12}$-alkyl, -CO-phenyl, -CO-pyridyl or -COO-$C_1$-$C_8$-alkyl-phenyl, whilst in the abovementioned group the phenyl ring may be substituted by $C_1$-$C_4$-alkyl, $C_1$-$C_4$-alkoxy, OH, halogen or $CF_3$,

with the proviso that compounds of formula II wherein

$R^3$ denotes a $C_1$-$C_5$-alkyl, phenyl- $C_1$-$C_3$-alkyl, $C_3$-$C_6$-cycloallcyl or pyridyl group and

$R^4$ denotes a hydrogen atom, a $C_1$-$C_5$-alkyl or phenyl- $C_1$-$C_3$-alkyl group

are excluded,
optionally in the form of the tautomers, the racemates, the enantiomers, the diastereomers and the mixtures thereof, and optionally the pharmacologically acceptable acid addition salts thereof.

**9.** Prodrugs of general formula (II) according to Claim 7, wherein

$R^1$ and $R^4$ may be as hereinbefore defined and

$R^3$ may have the meanings given above or denotes $C_1$-$C_4$-alkyl ,which is substituted by a group selected from among -C(=NOH)$NH_2$, -C(=NCOO-$C_1$-$C_6$-alkyl)$NH_2$ or -C(=NCOO-$C_1$-$C_6$-alkyl-phenyl)$NH_2$;

$R^7$ may denote hydroxy, -COO-$C_1$-$C_6$-alkyl, -CO-phenyl, -CO-pyridyl or -COO-$C_1$-$C_6$-alkyl-phenyl, whilst in the abovementioned group the phenyl ring may be substituted in each case by $C_1$-$C_4$-alkyl, $C_1$-$C_4$-alkoxy, OH, halogen or $CF_3$,

optionally in the form of the tautomers, the racemates, the enantiomers, the diastereomers and the mixtures thereof, and optionally the pharmacologically acceptable acid addition salts thereof.

**10.** Prodrugs of general formula (II) according to Claim 8 or 9, wherein

$R^1$, $R^3$ and $R^4$ may be as hereinbefore defined and

$R^7$ may denote hydroxy, methoxycarbonyl, ethoxycarbonyl, propyloxycarbonyl, butyloxycarbonyl,

benzoyl, benzyloxycarbonyl or nicotinoyl,

optionally in the form of the tautomers, the racemates, the enantiomers, the diastereomers and the mixtures thereof, and optionally the pharmacologically acceptable acid addition salts thereof.

**11.** Compounds of general formula (III)

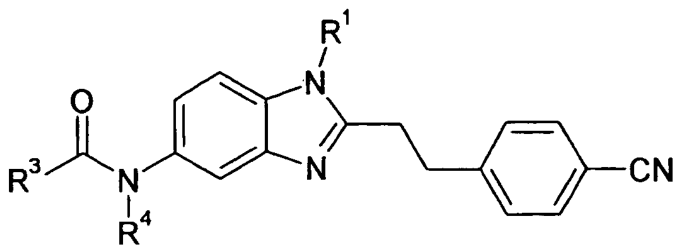

(III)

wherein the groups $R^1$, $R^3$ and $R^4$ may have the meanings given in claims 1-6, with the proviso that compounds of formula III wherein

$R^3$ denotes a $C_1$-$C_5$-alkyl, phenyl- $C_1$-$C_3$-alkyl, $C_3$-$C_6$-cycloalkyl or pyridyl group and

$R^4$ denotes a hydrogen atom, a $C_1$-$C_5$-alkyl or phenyl- $C_1$-$C_3$-alkyl group

are excluded.

**12.** Use of compounds of general formula (I) according to one of claims 1-6 for preparing a pharmaceutical composition for preventing and/or treating diseases in which tryptase inhibitors may provide a therapeutic benefit.

**13.** Use of prodrugs of general formula (II) according to one of claims 8, 9 or 10 for preparing a pharmaceutical composition for preventing and/or treating diseases in which tryptase inhibitors may provide a therapeutic benefit.

**14.** Pharmaceutical composition, **characterised in that** it contains one or more compounds according to one of claims 1-10.

**15.** Process for preparing compounds of general formula (I)

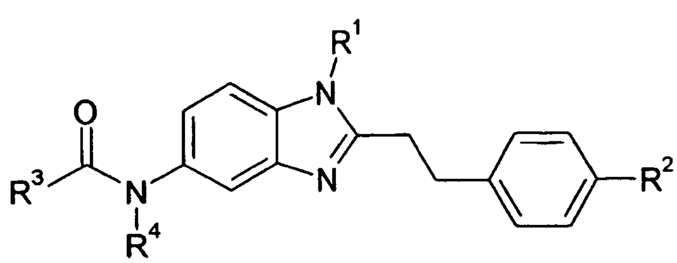

(I),

wherein the groups $R^1$, $R^2$, $R^3$ and $R^4$ may have the meanings given in claims 1-6, with the proviso that compounds of formula I wherein

$R^3$ denotes a $C_1$-$C_5$-alkyl, phenyl- $C_1$-$C_3$-alkyl, $C_3$-$C_6$-cycloalkyl or pyridyl group and

$R^4$ denotes a hydrogen atom, a $C_1$-$C_5$-alkyl or phenyl- $C_1$-$C_3$-alkyl group

are excluded,
**characterised in that** a compound of general formula (III)

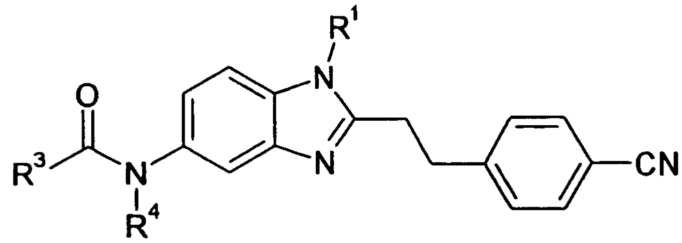

(III)

wherein the groups $R^1$, $R^3$ and $R^4$ may have the meanings given in claims 1-6, is converted directly into a compound of general formula (I).

## Revendications

**1.** Composés de formule générale (I)

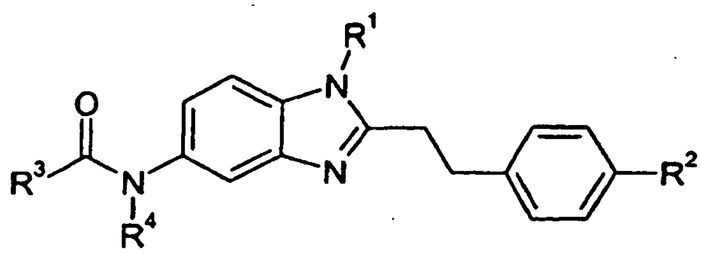

(I),

où
$R^1$ peut représenter $C_1$-$C_6$-alkyle,
$R^2$ peut représenter -C(=NH)$NH_2$
$R^3$ peut représenter un groupement $C_1$-$C_6$-alkyle, $C_1$-$C_6$-hydroxyalkyle ou $C_1$-$C_4$-alcoxy-$C_1$-$C_4$-alkyle, qui peut être substitué une ou deux fois par un, deux ou trois des groupements -$NR^5R^6$, -C(=NH)$NH_2$ ou -NH-C(=NH)$NH_2$, ou un hétérocycle saturé ou insaturé à 5, 6 ou 7 chaînons lié directement ou par le biais d'un pont $C_1$-$C_4$-alkylène ou d'un pont $C_2$-$C_4$-alcénylène, qui peut contenir un, deux ou trois hétéroatomes choisis dans le groupe oxygène, azote ou soufre, et qui peut éventuellement être substitué une ou deux fois par hydroxyle, $C_1$-$C_4$-alkyle, -COO-$C_1$-$C_4$-alkyle, -$CONH_2$, benzyle, diphénylméthyle, phényle ou pyridylméthyle, pyridyle, et où le substituant phényle peut être substitué une, deux ou trois fois par un ou plusieurs des groupements choisis dans le groupe $C_1$-$C_4$-alkyle, $C_1$-$C_4$-alcoxy, halogène, $C_1$-$C_4$-alkyl-halogène, -$NH_2$, ou cyclopropyle, cyclobutyle, cyclopentyle ou cyclohexyle, qui peuvent être substitués dans chaque cas une ou deux fois par un ou deux des groupements -$NR^5R^6$, -C(=NH)$NH_2$ ou -NH-C(=NH)$NH_2$, ou
phényl-$C_1$-$C_4$-alkyle ou naphtyl-$C_1$-$C_4$-alkyle, qui peut éventuellement être substitué par -$NR^5R^6$ au niveau du pont alkylène et qui peut être substitué sur le cycle phényle dans chaque cas une ou deux fois par ou un deux des groupements -$NO_2$, -$NR^5R^6$, $C_1$-$C_4$-alkyl-$NR^5R^6$, -C(=NH)$NH_2$ ou -NH-C(=NH)$NH_2$,
$R^4$ peut représenter l'hydrogène ou $C_1$-$C_6$-alkyle, qui peut éventuellement être substitué une ou deux fois par un ou deux groupements choisis dans le groupe furanyle, benzofuranyle, thiophényle, benzothiophényle, anthracényle, phényle, pyridyle et naphtyle, où les substituants phényle et naphtyle peuvent être substitués quant à eux dans chaque cas une, deux ou trois fois par un ou plusieurs des groupements choisis dans le groupe $C_1$-$C_4$-alkyle, $C_1$-$C_4$-alcoxy, halogène, $C_1$-$C_4$-alkyl-halogène, -$NH_2$, -NH($C_1$-$C_4$-alkyle), -N($C_1$-$C_4$-alkyle)$_2$, $NO_2$, hydroxyle, -$CF_3$, -NHCO-$C_1$-$C_4$-alkyle, -COOH, -COO($C_1$-$C_4$-alkyle), -$CONH_2$, -CONH($C_1$-$C_4$-alkyle), -CON($C_1$-$C_4$-alkyle)$_2$, -CONH($C_1$-$C_4$-alkyl)-COO($C_1$-$C_4$-alkyle) et phényl-$C_1$-$C_6$-alkyle ;
$R^5$ et $R^6$, identiques ou différents, peuvent représenter l'hydrogène, $C_1$-$C_4$-alkyle, phényle, pyridyle ou benzyle, qui peut éventuellement être substitué par un groupement choisi dans le groupe halogène, halogéno-$C_1$-$C_4$-alkyle, -OH, $C_1$-$C_4$-alkyle, -O-$C_1$-$C_4$-alkyle, -CO-O-$C_1$-$C_4$-alkyle, -$NO_2$, phényle, pyrrolidin-1-yle, pipéridin-1-yle, -$NH_2$, -NH-$C_1$-$C_4$-alkyle, -N($C_1$-$C_4$-alkyle)$_2$ et -C(=NH)$NH_2$,

avec la condition que les composés de formule I où
$R^3$ représente un groupe $C_1$-$C_5$-alkyle, phényl-$C_1$-$C_3$-alkyle, $C_3$-$C_6$-cycloalkyle, pyridyle, et
$R^4$ représente un atome d'hydrogène, un groupe $C_1$-$C_5$-alkyle ou phényl-$C_1$-$C_3$-alkyle
sont exclus, éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**2.** Composés de formule générale (I) selon la revendication 1, où
$R^1$ peut représenter $C_1$-$C_6$-alkyle,
$R^2$ peut représenter -C(=NH)$NH_2$ ;
$R^3$ peut représenter un groupement $C_1$-$C_6$-alkyle, qui peut être substitué une ou deux fois par un ou deux des groupements -$NR^5R^6$, -C(=NH)$NH_2$ ou -NH-C(=NH)$NH_2$, ou
un hétérocycle saturé ou insaturé à 5, 6 ou 7 chaînons lié directement ou par le biais d'un pont $C_1$-$C_4$-alkylène, qui peut contenir un, deux ou trois hétéroatomes choisis dans le groupe oxygène, azote ou soufre, et qui peut éventuellement être substitué une ou deux fois par hydroxyle, $C_1$-$C_4$-alkyle, benzyle, phényle ou pyridyle, et où le substituant phényle peut être substitué par l'un des groupements choisis dans le groupe $C_1$-$C_3$-alkyle, $C_1$-$C_3$-alcoxy, halogène, trifluorométhyle et - $NH_2$, ou
cyclopropyle, cyclopentyle ou cyclohexyle, qui peuvent être substitués dans chaque cas une ou deux fois par un ou deux des groupements $NR^5R^6$, -C(=NH)$NH_2$ ou -NH-C(=NH)$NH_2$, ou
phényl-$C_1$-$C_4$-alkyle ou naphtyl-$C_1$-$C_4$-alkyle, qui peut éventuellement être substitué par -$NR^5R^6$ au niveau du pont alkylène et qui peut être substitué sur le cycle phényle dans chaque cas une ou deux fois par ou un deux des groupements -$NO_2$, -$NR^5R^6$, $C_1$-$C_4$-alkyl-$NR^5R^6$, -C(=NH)$NH_2$ ou -NH-C(=NH)$NH_2$,
$R^4$ peut représenter l'hydrogène ou $C_1$-$C_6$-alkyle, qui peut éventuellement être substitué une ou deux fois par un ou deux groupements choisis dans le groupe phényle, pyridyle et naphtyle, où les substituants phényle et naphtyle peuvent être substitués quant à eux dans chaque cas par l'un des groupements choisis dans le groupe $C_1$-$C_4$-alkyle, $C_1$-$C_4$-alcoxy, halogène, $C_1$-$C_4$-alkyl-halogène, -$NH_2$;
$R^5$ et $R^6$, identiques ou différents, peuvent représenter l'hydrogène, $C_1$-$C_4$-alkyle, pyridyle ou benzyle, qui peut éventuellement être substitué par un groupement choisi dans le groupe -OH, -O-$C_1$-$C_3$-alkyle, -$NO_2$, phényle, pyrrolidin-1-yle, -$NH_2$, -NH-$C_1$-$C_4$-alkyle, -N($C_1$-$C_4$-alkyle)$_2$ et -C(=NH)$NH_2$,
éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**3.** Composés de formule générale (I) selon la revendication 1 ou 2, où
$R^1$ peut représenter $C_1$-$C_4$-alkyle,
$R^2$ peut représenter -C(=NH)$NH_2$ ;
$R^3$ peut représenter un groupement $C_1$-$C_4$-alkyle, qui peut être substitué une ou deux fois par un ou deux des groupements -$NR^5R^6$, -C(=NH)$NH_2$ ou -NH-C(=NH)$NH_2$, ou
un hétérocycle saturé ou insaturé à 5, 6 ou 7 chaînons lié directement ou par le biais d'un pont méthylène ou éthylène, qui peut contenir un ou deux hétéroatomes choisis dans le groupe oxygène ou azote, et qui peut éventuellement être substitué par méthyle ou benzyle;
naphtylméthyle, benzyle ou phényléthyle, qui peuvent éventuellement être substitués par -$NR^5R^6$ au niveau du pont alkylène et qui peuvent être substitués sur le cycle phényle par un groupement choisi dans le groupe -$NR^5R^6$, -$C_1$-$C_4$-alkyl-$NR^5R^6$, -C(=NH)$NH_2$ et -NH-C(=NH)$NH_2$,
$R^4$ peut représenter l'hydrogène ou $C_1$-$C_5$-alkyle, qui peut éventuellement être substitué une ou deux fois par un ou deux groupements choisis dans le groupe phényle, pyridyle et naphtyle ;
$R^5$ et $R^6$, identiques ou différents, peuvent représenter l'hydrogène, méthyle, éthyle, propyle, butyle, pyridyle ou benzyle, qui peut éventuellement être substitué par un groupement choisi dans le groupe -OH, méthoxy, -$NO_2$, phényle, pyrrolidin-1-yle, -$NH_2$, -NH-méthyle, -N(méthyle)$_2$, -NH-éthyle, -N(éthyle)$_2$ et - C(=NH)$NH_2$,
éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**4.** Composés de formule générale (I) selon la revendication 1, 2 ou 3, où
$R^1$ peut représenter méthyle, éthyle, propyle ou butyle ;
$R^2$ peut représenter -C(=NH)$NH_2$ ;
$R^3$ peut représenter un groupement $C_2$-$C_4$-alkyle, qui peut être substitué une ou deux fois par un ou deux des groupements -$NR^5R^6$, -C(=NH)$NH_2$ ou -NH-C(=NH)$NH_2$, ou

un hétérocycle saturé ou insaturé à 6 chaînons lié par le biais d'un pont méthylène ou éthylène, qui contient un ou deux atomes d'azote et qui peut éventuellement être substitué par méthyle ou benzyle ;
naphtylméthyle, benzyle ou phényléthyle, qui peuvent éventuellement être substitués par $-NR^5R^6$ au niveau du pont alkylène et qui sont substitués sur le cycle phényle par un groupement choisi dans le groupe $-NR^5R^6$, $-C_1-C_4$-alkyl-$NR^5R^6$, $-C(=NH)NH_2$ et $-NH-C(=NH)NH_2$,
$R^4$ peut représenter l'hydrogène ou un groupement alkyle choisi dans le groupe méthyle, éthyle, propyle, butyle et pentyle, qui peut éventuellement être substitué une ou deux fois par un ou deux groupements choisis dans le groupe phényle, pyridyle et naphtyle ;
$R^5$ et $R^6$, identiques ou différents, peuvent représenter l'hydrogène, méthyle, éthyle, propyle, butyle, pyridyle ou benzyle, qui peut éventuellement être substitué par un groupement choisi dans le groupe -OH, méthoxy, $-NO_2$, phényle, pyrrolidin-1-yle, $-NH_2$, -NH-méthyle, -N(méthyle)$_2$ et $-C(=NH)NH_2$,
éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**5.** Composés de formule générale (I) selon l'une quelconque des revendications 1, 2, 3 ou 4, où
$R^1$ peut représenter méthyle, éthyle ou propyle;
$R^2$ peut représenter $-C(=NH)NH_2$ ;
$R^3$ peut représenter méthyle, qui est substitué par un groupement choisi dans le groupe pyridylamino, benzylamino, N-benzyl-N-méthylamino, N-(amidinobenzyl)amino, N-(amidinobenzyl)-N-méthyl-amino, N-(diméthylaminobenzyl)-amino, (pyrrolidin-1-ylbenzyl)amino et N-(diméthylaminobenzyl)-N-méthylamino, ou
un groupement alkyle choisi dans le groupe éthyle et propyle, qui peut être substitué une ou deux fois par un ou deux groupements choisis dans le groupe $-NH_2$ et $-NH-C(=NH)NH_2$, ou
un hétérocycle lié par le biais d'un pont éthylène choisi dans le groupe pipéridine, morpholine et pipérazine, qui peut éventuellement être substitué par méthyle, benzyle ou diphénylméthyle ;
phényléthyle, qui peut éventuellement être substitué par $-NH_2$ au niveau du pont éthylène et qui est substitué sur le cycle phényle par un groupement choisi dans le groupe pyrrolidin-1-yle, $-NH_2$, -N(méthyle)$_2$, $-CH_2-NH_2$ et $-C(=NH)NH_2$ ;
$R^4$ peut représenter l'hydrogène, méthyle, éthyle, propyle, butyle, pentyle, benzyle, naphtalinylméthyle, pyridylméthyle ou diphénylpropyle ;
éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**6.** Composés de formule générale (I) selon l'une des revendications 1, 2, 3, 4 ou 5, où
$R^1$ peut représenter méthyle, éthyle ou propyle;
$R^2$ peut représenter $-C(=NH)NH_2$ ;
$R^3$ peut représenter méthyle, qui est substitué par un groupement choisi dans le groupe N-(amidinobenzyl)amino, N-(amidinobenzyl)-N-méthyl-amino, N-(diméthylaminobenzyl)amino, (pyrrolidin-1-ylbenzyl)amino et N-(diméthylaminobenzyl)-N-méthyl-amino, ou
une pipéridine ou pipérazine liée par le biais d'un pont éthylène, qui peut éventuellement être substituée par benzyle ;
phényléthyle, qui peut éventuellement être substitué par $-NH_2$ au niveau du pont éthylène et qui est substitué sur le cycle phényle par $-C(=NH)NH_2$ ;
$R^4$ peut représenter l'hydrogène, méthyle, butyle, benzyle, naphtalinylméthyle ou diphénylpropyle, éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**7.** Composés de formule générale (IA)

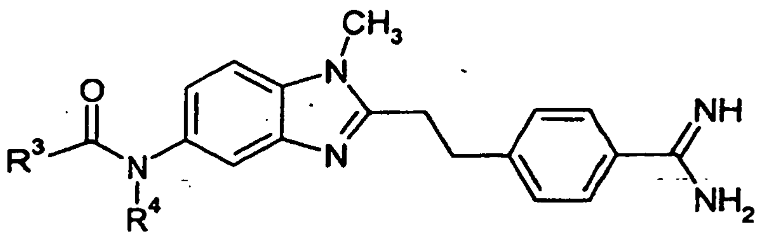

(IA),

où

$R^3$ peut représenter méthyle, qui est substitué par un groupement choisi dans le groupe N-(amidinobenzyl)amino, N-(amidinobenzyl)-N-méthyl-amino, N-(diméthylaminobenzyl)amino, (pyrrolidin-1-ylbenzyl)amino et N-(diméthylaminobenzyl)-N-méthyl-amino, ou

une pipéridine ou pipérazine liée par le biais d'un pont éthylène, qui peut éventuellement être substituée par benzyle ;

phényléthyle, qui peut éventuellement être substitué par $-NH_2$ au niveau du pont éthylène et qui est substitué sur le cycle phényle par $-C(=NH)NH_2$ ;

$R^4$ peut représenter l'hydrogène, méthyle, butyle, benzyle, naphtalinylméthyle ou diphénylpropyle, éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**8.** Promédicaments de formule générale (II)

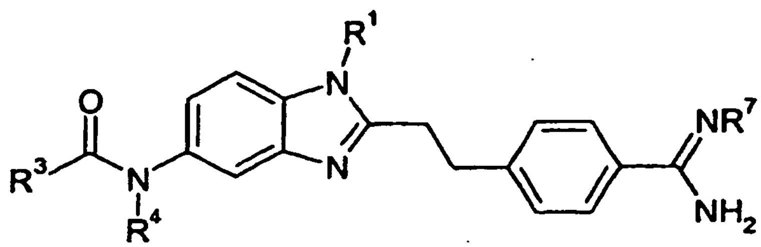

(II),

où

$R^1$ et $R^4$ peuvent présenter les significations citées précédemment et

$R^3$ peut présenter les significations citées précédemment ou représente $C_1$-$C_4$-alkyle qui est substitué par un groupement choisi dans le groupe $-C(=NOH)NH_2$, $-C(=NCOO-C_1-C_{12}\text{-alkyl})NH_2$ ou $-C(=NCOO-C_1-C_8\text{-alkyl-phényl})NH_2$ ;

$R^7$ peut représenter hydroxyle, $-COO-C_1-C_{12}$-alkyle, -CO-phényle, -CO-pyridyle ou $-COO-C_1-C_8$-alkyl-phényle, où dans le groupe cité précédemment le cycle phényle peut être substitué à chaque fois par $C_1$-$C_4$-alkyle, $C_1$-$C_4$-alcoxy, OH, halogène ou $CF_3$,

avec la condition que les composés de formule II où

$R^3$ représente un groupe $C_1$-$C_5$-alkyle, phényl-$C_1$-$C_3$-alkyle, $C_3$-$C_6$-cycloalkyle, pyridyle, et

$R^4$ représente un atome d'hydrogène, un groupe $C_1$-$C_5$-alkyle ou phényl-$C_1$-$C_3$-alkyle sont exclus, éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**9.** Promédicaments de formule générale (II) selon la revendication 8 où

$R^1$ et $R^4$ peuvent présenter les significations citées précédemment et

$R^3$ peut présenter les significations citées précédemment ou représente $C_1$-$C_4$-alkyle qui est substitué par un groupement choisi dans le groupe $-C(=NOH)NH_2$, $-C(=NCOO-C_1-C_6\text{-alkyl})NH_2$ ou $-C(=NCOO-C_1-C_6\text{-alkyl-phényl})NH_2$ ;

$R^7$ peut représenter hydroxyle, $-COO-C_1-C_6$-alkyle, -CO-phényle, -CO-pyridyle ou $-COO-C_1-C_6$-alkyl-phényle, où dans le groupe cité précédemment le cycle phényle peut être substitué à chaque fois par $C_1$-$C_4$-alkyle, $C_1$-$C_4$-alcoxy, OH, halogène ou $CF_3$, éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**10.** Promédicaments de formule générale (II) selon la revendication 8 ou 9 où
$R^1$, $R^3$ et $R^4$ peuvent présenter les significations citées précédemment et
$R^7$ peut représenter hydroxyle, méthoxycarbonyle, éthoxycarbonyle, propyloxycarbonyle, butyloxycarbonyle, benzoyle, benzyloxycarbonyle ou nicotinoyle, éventuellement sous forme de leurs tautomères, de leurs racémates, de leurs énantiomères, de leurs diastéréoisomères et de leurs mélanges, et éventuellement de leurs sels d'addition d'acide pharmacologiquement acceptables.

**11.** Composés de formule générale (III)

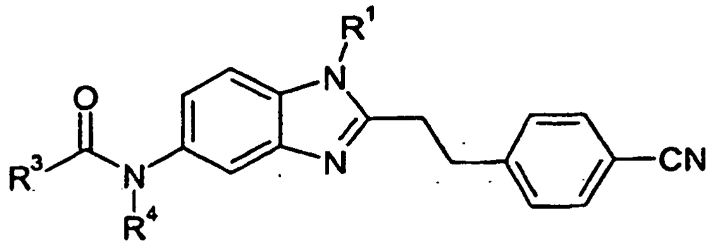

**(III)**

où les groupements $R^1$, $R^3$ et $R^4$ peuvent présenter les significations citées dans les revendications 1-6, avec la condition que les composés de formule III où
$R^3$ représente un groupe $C_1$-$C_5$-alkyle, phényl-$C_1$-$C_3$-alkyle, $C_3$-$C_6$-cycloalkyle, pyridyle, et
$R^4$ représente un atome d'hydrogène, un groupe $C_1$-$C_5$-alkyle ou phényl-$C_1$-$C_3$-alkyle sont exclus.

**12.** Utilisation de composés de formule générale (I) selon l'une des revendications 1-6 pour la production d'un médicament pour la prévention et/ou le traitement des maladies dans lesquelles les inhibiteurs de tryptase peuvent présenter une utilité thérapeutique.

**13.** Utilisation de promédicaments de formule générale (II) selon l'une des revendications 8, 9 ou 10 pour la production d'un médicament pour la prévention et/ou le traitement des maladies dans lesquelles les inhibiteurs de tryptase peuvent présenter une utilité thérapeutique.

**14.** Composition pharmaceutique **caractérisée par** une teneur en un ou plusieurs composés selon l'une des revendications 1-10.

**15.** Procédé de préparation de composés de formule générale (I)

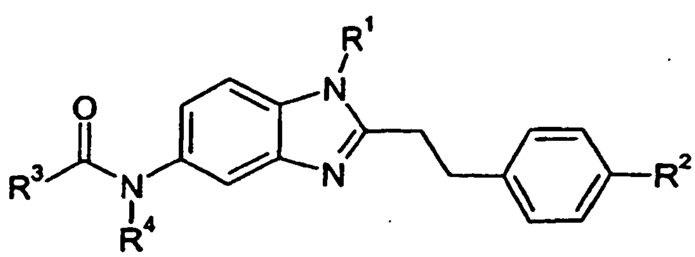

**(I),**

où les groupements $R^1$, $R^2$, $R^3$ et $R^4$ peuvent présenter les significations citées dans les revendications 1-6, avec la condition que les composés de formule I où
$R^3$ représente un groupe $C_1$-$C_5$-alkyle, phényl-$C_1$-$C_3$-alkyle, $C_3$-$C_6$-cycloalkyle, pyridyle, et
$R^4$ représente un atome d'hydrogène, un groupe $C_1$-$C_5$-alkyle ou phényl-$C_1$-$C_3$-alkyle sont exclus, **caractérisé en ce qu'**un composé de formule générale (III)

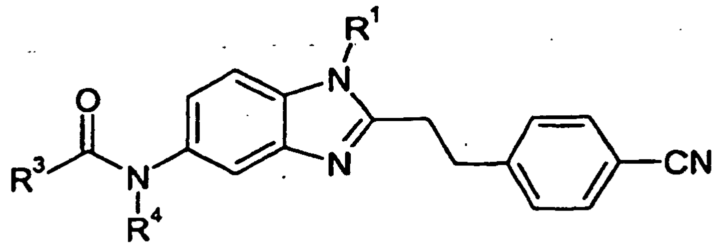

(III)

où les groupements $R^1$, $R^3$ et $R^4$ peuvent avoir les significations citées dans les revendications 1-6 est converti directement en les composés de formule générale (I).